# EUROPEAN PATENT APPLICATION

(11) **EP 2 490 021 A1**
(43) Date of publication of application: **22.08.2012**
(21) Application number: 11155096.8
(22) Date of filing: 18.02.2011
(51) Int. Cl.: G01N 33/50, G01N 33/569, A61K 31/00

(54) **Modulators of PRR and GPCR signalling**

(71) Applicant: Biotempt B.V., 7958 NZ Koekange (NL)
(72) Inventor: The designation of the inventor has not yet been filed
(74) Representative: Jansen, Cornelis Marinus

(57) **Abstract**

The invention relates to methods for determining whether a peptide is capable of modulating signalling via at least one Pattern Recognition Receptor (PRR) signalling pathway and/or G protein coupled receptor (GPCR) signalling pathway, the method comprising providing a cell susceptible to signalling through at least one of said pathways, contacting said cell with at least one dipeptide, contacting said cell with an agonistic or antagonistic compound of a PRR and/or a GPCR of said pathway, and determining the activity of said at least one signalling pathway in said cell. The invention further relates to use of peptides in therapeutic applications where modulation of at least one PRR and/or GPCR signalling pathway is desirable.

## Description

The invention relates generally to biotechnology and medicine. The invention further relates generally to methods of identifying lead molecules that have an increased probability of becoming a medicament, useful, for example, in modulating the innate immunity and inflammatory response in a subject. The invention can be used, *inter alia,* for research, diagnostic and/or therapeutic products, methods and devices.

Inflammation in a subject can be the result of tissue damage following mechanical or chemical irritation, a reaction to infections, or the result of abnormal immunological reactions (*e.g*., autoimmune diseases, chronic inflammatory diseases). Acute inflammation is halted by elimination of the provoking factor; *i.e*., lysed cells in the case of tissue damage, or pathogens in the case of infections. Chronic inflammation occurs when the provoking event cannot be eliminated, or if the immune response becomes independent, for example, an autoimmune disease develops, in which the immune response is directed against autoantigens or against the ubiquitous intestinal flora.

A central event of inflammatory reactions is leukocyte migration into the inflammatory focus. The recruited leukocytes release proinflammatory substances; *e.g*., substances that increase permeability, and toxic substances, or trigger the release of such substances by other cells (for example, cytokines, enzymes, mediators, prostaglandins, oxygen radicals, etc.). This leads to increased perfusion and to the activation and increased output of immunocytes, which are involved in the elimination of the pathogens and removal of decomposition products. Various growth factors, including cytokines, promote the proliferation of tissue cells and thus the healing process.

The detection of pathogens by the host is achieved through the families of pattern recognition receptors (PRR) that recognize conserved molecular structures known as pathogen-associated molecular patterns (PAMP) and induce production of innate effector molecules. (Welss et al, Published online before print September 8, 2010, doi: 0.1073/pnas.1000092107 *PNAS September 8, 2010)* Because these structures are also found on nonpathogenic microorganisms or on host tissue, the terms microbe-associated molecular patterns (MAMP) or damage-associated molecular patterns (DAMP)are increasingly used, particularly in the context of host-commensal interactions or host-host interactions. These signalling receptors can be divided into at least four families that currently are in the centre of interest of those studying innate immunity: Toll-like receptors (TLR), retinoic acid inducible gene I (RIG-I)-like receptors (RLR), nucleotide oligomerization domain (NOD)-like receptors (NLR) and formyl-peptide receptors (FPR). Other PPR familie members, such as the predominantly myeloid cell-expressed C-type lectin-like receptor dectin-1 which is a pattern-recognition receptor that recognizes β1,3-linked glucans found in fungi, plants and some bacteria, as well as an unidentified ligand on *Mycobacteria* sp, deserve further characterization.

The TLR family is the best characterized and 13 receptors have been reported in mice and humans. For TLR, it has been shown that ligand sensing and specificity is achieved through the arrangement and sequence variation in the conserved leucine-rich repeat (LRR) domains. TLR2 can form heterodimers with TLR1 or TLR6 to detect different but related ligands. TLR are localized in the cell membrane and/or endosomal membrane components to recognize extracellular and endocytosed MAMP. Toll-like receptors (TLRs) are a family of membrane proteins involved in the innate immune system that recognize danger signals in the form of conserved motifs of necessary molecules derived from pathogens. Once these pathogens have entered the body through the skin or mucosa, the conserved motifs presented by the pathogens are recognized by TLRs, which activate an immune response. TLRs are "pattern recognition receptors" (PRRs), and thus recognize molecules that are broadly shared by pathogens, but that are distinguishable from host molecules. This class of pathogen molecules is collectively referred to as pathogen-associated molecular patterns (PAMPs). Thirteen TLRs (referred to as TLR1 through TLR13, respectively) have been identified in humans and mice, and homologues of many of these have been identified in other mammalian species. Well-conserved features in pathogens that are recognized by particular TLRs include bacterial cell-surface lipopolysaccharide (LPS), lipoproteins, lipopeptides and lipoarabinomannan; proteins such as flagellin from bacterial flagella; double-stranded RNA of viruses or the unmethylated CpG islands of bacterial and viral DNA; and certain other RNA and DNA. In addition to the recognition of conserved microbial products which signal the presence of an infection and trigger the cellular immune response, TLRs also detect some endogenous ligands that might signal other danger conditions, such as degradation products of macromolecules, products of proteolytic cascades, intracellular components of ruptured cells and products of genes that are activated by inflammation. See , e.g., Zhang and Schluesener (2006) Cell. Moll. Life Sci (63) 2901-7. For most TLRs, ligand recognition specificity has now been established by gene targeting. See, *e.g.,* Hoebe et al. (2003) Nature 424(6950):743-8; Hemmi et al. (2000) Nature 408(6813):740-5. Databases of Toll-like receptors and their ligands exist, for example, on the Internet at zeus.krist.geo.uni-muenchen.de/~tollml/.

TLR activation provokes a stereotypic inflammatory response, and thus endogenous activators of TLRs may participate in autoimmune diseases. TLRs have been suspected of binding to a variety of host molecules, including fibrinogen, heat shock proteins, and host DNA. When activated, TLRs recruit adapter molecules within the cytoplasm of cells. At least four adapter molecules are known to be involved in TLR-mediated signalling; MyD88, TIRAP (Ma1), TRIF, and TRAM. The adapters activate other signalling molecules within the cell, including certain protein kinases (IRAK1, IRAK4, TBK1, and IKKi) that amplify the signal, and ultimately lead to the induction or suppression of genes that orchestrate the inflammatory response. TLR4 is a TLR that recognizes numerous pathogen molecules, *e.g*., LPS from Gram-negative bacteria, and several heat shock proteins from bacteria. In addition, TLR4 may recognize several host molecules, including host heat shock proteins, fibrinogen, heparan sulfate fragments, and hyaluronic acid fragments. Signalling via Toll like receptors that influences the inflammatory response via both cell survival pathways, for instance involving PKB/AKT, and inflammatory cytokine pathways, for instance involving ERK, JNK and p38 MAPK.

For the purpose of ease of definition herein, in this application in the phrase "TLR signalling pathways" , both the IL-1R and the traditional TLR signalling pathways are included In the TLR signalling pathways, following stimulation and dimerisation, the various IL-1R and TLR signalling pathways, with the exception of TLR3, recruit the adaptor molecule MyD88 and induce nuclear factor (NF)-κB and mitogen-activated protein kinases (MAPKs) through IL-1R associated kinase (IRAK)-4, IRAK-1 and TNF receptor associated factor (TRAF)-6. In addition, a MyD88-independent signalling pathway is utilised by TLR3 and TLR4, which depends on the adaptor molecule TRIF (TIR-domain-containing adapter-inducing interferon-β) and leads to the induction of interferon regulatory factors (IRFs) and a late activation of NF-κB. Signalling through TLR4 results in phosphoprylation and activation of protein tyrosine kinases (TKs). The Tec family member Btk interacts with the Toll/IL-1 receptor (TIR) domains ofTLRs, MyD88 and Mal (MyD88 adaptor like protein). Once activated, Btk phoshporylates Mal and activates NF-κB and/or p38 MAPK. Src family kinases (SFKs; for example, Hck) are known to function upstream of both Pyk2 and Syk kinases, respectively, in TLR signalling. TLRs mediate phosphatidylinositol-3-kinase (PI3K) activation that suppresses p38 MAPK and NF-κB. Inhibition of these signalling cascades by PI3K is mediated by protein kinase B (PKB), and limits the production of inflammatory cytokines.

The ubiquitously expressed RIG-I-like receptor (RLR) family of RNA helicases are cytoplasmic proteins that recognize viral RNAs and induce innate antiviral responses, including the activation of proinflammatory cytokines and type I interferon (IFN). Examples of RLRs are RIG-I (retinoic-acid-inducible protein 1, also known as Ddx58) and MDA-5 (melanoma-differentiation-associated gene 5, also known as Ifih1 or Helicard).

The third family, containing >20 cytoplasmic NLR in humans and mice, is divided into four major groups based on the nature of the N-terminal activation domains involved in signal transduction. The NLR characterized to date recognize certain damage-associated molecular patterns (DAMP) of the host cell. NLR proteins can signal through different multicomponent signal complexes to activate alternative signalling pathways, including caspase activation, cell death, and NF-κB leading to cytokine, chemokine, and defensin expression. The NLRs are cytoplasmic proteins that may have a variety of functions in regulation of inflammatory and apoptotic responses. NLRs are encoded by genes from a large gene family present in many different animal species; there are more than 20 NLR genes in humans. Many are thought to serve as pattern recognition receptors (PRRs) which sense microbial products in the cytoplasm of cells, although some members have different functions. New terminology for NOD-like receptors was adopted by the Human Genome Organization (HUGO) in 2008 to standardize confusing nomenclature of NLRs. The acronym NLR, once standing for NOD-like receptor, now serves as an abbreviation for is "nucleotide-binding domain, leucine-rich repeat containing" protein since these proteins are composed of conserved "modules" including a central nucleotide-binding oligomerization domain and a series of tandem leucine-rich repeats. NLRs are also known as CATERPILLERs. Although the ligands and functions of many of these receptors are not known, their primary role is to recognize cytoplasmic pathogen-associated molecular patterns (PAMPs) and/or endogenous danger signals also called damage-associated molecular patterns (DAMPs), inducing immune responses. NLRs are characterized by a tripartite-domain organization with a conserved nucleotide binding oligomerization domain (NOD) and leucine-rich repeats (LRRs). The general domain structure consists of C-terminal LRRs involved in microbial sensing, a centrally located NOD domain and an N-terminal effector region comprising a protein-protein interaction domain such as the CARD, Pyrin or BIR domain. NLRs have been grouped into several subfamilies on the basis of their effector domains: NODs, NALPs, CIITA, IPAF, and NAIPs. NODs and IPAF contain CARD effector domains, whereas NALPs and NAIPs contain pyrin (PYD) effector domains and three BIR domains, respectively. The first mammalian NLRs reported to sense intracellular microbial PAMPs are NOD1 (CARD4) and NOD2 (CARD15), which contain one and two N-terminal CARD domains, respectively. They recognize peptidoglycan (PGN), an essential constituent of the bacterial cell wall. NOD1 and NOD2 detect specific motifs within the PGN. NOD1 senses the D-γ-glutamyl-meso-DAP dipeptide (iE-DAP) which is found in PGN of all Gram-negative and certain Gram-positive bacteria whereas NOD2 recognizes the muramyl dipeptide (MDP) structure found in almost all bacteria. Thus NOD2 acts as a general sensor of PGN and NOD1 is involved in the recognition of a specific subset of bacteria. Both iE-DAP and MDP must be delivered intracellularly either by bacteria that invade the cell or through other cellular uptake mechanisms to be detected by NOD1 and NOD2 respectively. NOD1 and NOD2 signal via the serine/threonine RIP2 (RICK,CARDIAK) kinase through CARD-CARD homophilic interactions. Once activated, RIP2 mediates ubiquitination of NEMO/IKKγ leading to the activation of NF-κB and the production of inflammatory cytokines such as TNF-α and IL-6. In addition to the NF-κB pathway, NOD1 and NOD2 stimulation induces the activation of MAPKs. Recent work has implicated CARD9 in the selective control of NOD2-dependent p38 and JNK signalling. The physiological importance of NOD1 and NOD2 in immune responses is evident from the linkage of their mutations with inflammatory diseases in humans. Genetic variation in NOD2 is associated with Crohn's disease, one of the major forms of inflammatory bowel diseases. Several NOD1 polymorphisms are linked to the development of atopic eczema and asthma.

Formyl peptide receptors (FPRs) are a small group of 7-transmembrane domain, G protein-coupled receptors that are expressed mainly by mammalian phagocytic leukocytes and known to be important in host defense and inflammation. The three human FPRs (FPR1, FPR2/ALX and FPR3) share significant sequence homology and are encoded by clustered genes. Collectively, these receptors bind an extraordinarily numerous and structurally diverse group of agonistic ligands, including N-formyl and non-formal peptides of different composition, that chemoattract and activate phagocytes. N-formyl peptides, which are encoded in nature only by bacterial and mitochondrial genes and result from obligatory initiation of bacterial and mitochondrial protein synthesis with N-formylmethionine, is the only ligand class common to all three human receptors.

FPR's are G protein-coupled receptors (GPCRs). GPCRs form a large and diverse family of proteins whose primary function is to transduce extracellular stimuli into intracellular signals and are also called 7-TM receptors or the heptahelical receptors. GPCRs are characterized by seven transmembrane segments. This class of membrane proteins can respond to a wide range of agonists, including photon, amines, hormones, neurotransmitters, light-sensitive compounds, odors, pheromones and other proteins and vary in size from small molecules to peptides to large proteins. Some agonists bind to the extracellular loops of the receptor, others may penetrate into the transmembrane region. G protein-coupled receptors are involved in many diseases, and are also the target of approximately 30% of all modern medicinal drugs. The first step in GPCR signal transduction is ligand binding. Depending on the type of G protein to which the receptor is coupled, a variety of downstream signalling pathways can be activated. Central in innate immunity stand chemokines. Chemokine receptors comprise a large family of seven transmembrane domain G protein-coupled receptors differentially expressed in diverse cell types. Biological activities have been most clearly defined in leukocytes, where chemokines coordinate development, differentiation, anatomic distribution, trafficking, and effector functions and thereby regulate innate and adaptive immune responses. Evolution selected GPCR-based systems to serve three of our sensory functions.Vision, taste, and smell all rely on GPCRs. In case of olphactory detection, single receptors can engage several different odorants, and a single odorant can bind several different receptors. By combinatorial engagement of approximately 400 different odorant receptors, humans can distinguish several thousand different odors. Similarly, chemokine receptors fill a sensory function for the immune system.

The cytoplasmic parts of a GPCR protein (three intracellular loops and the C-terminal tail) can interact with a heterotrimeric, guanine nucleotide-binding protein (G-protein). The task of the G-protein is to transfer the ligand-induced signal into a variety of second messengers and expansion of downstream signalling. The G-protein is composed of three subunits, α, β and γ. The α unit has a binding site for GDP/GTP and possesses the GTPase activity that enables the transformation of GTP to GDP. Activation of the GPCR induces a conformational change in the receptor that is followed by an exchange of GDP for GTP bound to Gα. Subsequently, the G-protein dissociates into Gα- and Gβγ-subunits that proceed to activate or inhibit different signalling pathways. After passing the signal on to effectors elsewhere in the cell, bound GTP is hydrolyzed to GDP and the Gα subunit is recirculated to the membrane to participate in the formation a new G protein/receptor complex. Four main families of G-proteins have been characterized based on the classification of α subunits, G_{αs}, G_{αi/o}, G_{αq/11}, and G_{α12/13}. Examples of ligands for GPCRs include adenosine, bradykinin, endothelin, γ-aminobutyric acid (GABA), hepatocyte growth factor (HGF), melanocortins, neuropeptide Y, opioid peptides, opsins, somatostatin, tachykinins, members of the vasoactive intestinal peptide family, vasopressin, dopamine, epinephrine, norepinephrine, histamine, glutamate, glucagon, acetylcholine, serotonin, chemokines; lipid mediators of inflammation (e.g., prostaglandins, prostanoids, platelet-activating factor, and leukotrienes); and peptide hormones (e.g., calcitonin, C5a anaphylatoxin, follicle-stimulating hormone (FSH), gonadotropin-releasing hormone (GnRH), neurokinin, thyrotropin-releasing hormone (TRH), and oxytocin).

Lipopolysaccharides (LPS) have a lipid component, lipid A, and a polysaccharide unit covalently linked to this membrane domain. The polysaccharide region has the terminal O-specific chain, a substructure that comprises up to 50 repeating oligosaccharide units of usually two to eight monomers, and the core region, which is linked to the lipid A. Lipopolysaccharides are formed by gram-negative bacteria and are highly potent stimulators of innate immunity. They bind to Toll-like receptor 4 (TLR4)/CD14 receptors on human mononuclear cells and induce the formation and secretion of proinflammatory cytokines, such as TNF-α, MIF, IL-1beta, IL-6, IL-8, IL-12, IL-15, and IL-18; various colony-stimulating factors; various lipid mediators; and reduced oxygen species. In addition, LPS produces rapid-onset, antibody-dependent activation of the complement cascade with release of the anaphylatoxins C5a and C3a. Human T lymphocytes are stimulated to proliferate and to produce IL-2 and IFN. During the further course of the inflammatory reaction, the formation of acute phase proteins is induced. Lipopolysaccharides activate the nonspecific immune response by these mechanisms, and microorganisms, viruses, and tumour cells can be better eliminated as a result of this response. Phenomena such as inflammation, fever, and sepsis are induced by the LPS-induced production of cytokines and lipid mediators. Due to the toxic effect of LPS in sepsis, agonistic LPS are also called endotoxins.

It is an object of the present invention to provide methods for determining whether a peptide is capable of modulating signalling via at least one Pattern Recognition Receptor (PRR) signalling pathway and/or G protein coupled receptor (GPCR) signalling pathway.

The current invention relates to understanding and/or predicting the body's way of responding to small molecules and builds on insights reported in PCT International Publications WO99/59617 and WO01/72831 and PCT International Application PCT/NL02/00639, the contents of the entirety of all of which are incorporated herein by this reference. These applications describe small gene-regulatory peptides that are present in pregnant women and are derived from proteolytic breakdown of placental gonadotropins, such as hCG. These peptide products of proteolysis or breakdown products are often only about three to six amino acids long and were shown to have immunological activity that is exerted by regulating expression of genes encoding inflammatory mediators such as cytokines. Surprisingly, it was found that breakdown of hCG provides a cascade of peptides that helps maintain a pregnant woman's immunological homeostasis. These peptides balance the immune system to assure that the mother stays immunologically sound while her foetus does not get prematurely rejected during pregnancy, but instead is safely carried until its time of birth. Other peptides known in the art have the antigenic binding activity of human chorionic gonadotropin (hCG). *See, e.g.,* U.S. Patent 5,380,668 to Herron (Jan. 10, 1995), the contents of the entirety of which are incorporated by this reference. The oligopeptides disclosed therein are disclosed generally for use in diagnostic methods.

Other patents and patent applications to Gallo *et al*. (*e.g.,* U.S. Patent 5,677,275 (corresponding to WO 96/04008 A1), U.S. Patent 5,877,148 (also corresponding to WO 96/04008 A1), WO 97/49721 A1, U. S. Patent 6,319,504 (corresponding to WO 97/49373), U.S. Patent Application 2003/0049273 A1 (also corresponding to WO 97/49373), U.S. Patent 5,968,513 (corresponding to WO 97/49418), U.S. Patent 5,997,871 (corresponding to WO 97/49432), U.S. Patent 6,620,416, U.S. Patent 6,596,688, WO 01/11048 A2, WO 01/10907 A2., and U.S. Patent 6,583,109) relate to various oligopeptides and their use in, among other things, "inhibiting HIV infection," "treating or preventing HIV infection," "treating or preventing cancer," "treating or preventing a condition characterized by loss of body cell mass," "treating or preventing a condition associated with pathological angiogenesis," "treating or preventing hematopoietic deficiency," "*ex vivo* gene therapy," "expanding blood cells *in vitro*," and/or "providing blood cells to a subject."

Before the present invention the mechanism underlying the activity of the small gene-regulatory peptides that are present in pregnant women and are derived from proteolytic breakdown of placental gonadotropins, such as hCG, was unclear (Khan, N.A. et al. 2010, Clinical and Experimental Immunology, 160: 466-478, Khan, N.A. et al. 2009, Nephrol Dial Transplant, 24(9): 2701-2708, Van den Berg, H.R. et al. 2009, Shock, 31(3): 285-291). Van den Berg *et* al. stated that it is unclear what the underlying mechanism is by which these oligopeptides exert their effects, and that it is possible that they use yet unidentified receptors. Further, the authors mention that they do not exclude that different oligopeptides have different modes of action. Khan *et al*. (2009) state that it is unclear what the molecular mechanism of action is by which AQGV exerts its effects. The authors mention that it is possible that AQGV mediates its effect by an as yet unidentified receptor. Khan *et al*(2009) further propose that AQGV interferes specifically with pathways required for E-selectin transcriptional activation. E-selectin is expressed *de novo* on endothelial cells after transcriptional induction by pro-inflammatory agents. It is mentioned that it is not clear whether AQGV inhibits the local production of proinflammatory mediators that induce E-selectin or directly interferes with the intracellular signalling cascade involved in activating E-selectin transcription. Khan *et al.* (2010) also state that is unknown whether such small oligopeptides act by binding to surface-expressed or cytosolic receptors. The authors only exclude that LQGV, AQGV and VLPALP act via binding to the luteinizing hormone (LH) receptor as they do not bind to the LH receptor nor do they interfere with the binding of hCG to the LH receptor. Van den Berg *et al.*, Khan *et al*. (2009) and Khan *et al.* (2010) all propose the possibility that, due to their small size and molecular weight, the oligopeptides penetrate the cell membrane and exert their action either by interfering with signaling cascades or the transcriptional machinery.

Surprisingly, the present inventors discovered that peptides of 2-12 naturally occurring amino acids resulting from breakdown of hCG are potent regulators of Pattern Recognition Receptor (PRR) dependent signalling pathways and G protein-coupled receptor (GPCR) dependent signalling pathways. In Examples 1-3 it is shown that tetrapeptide AQGV modulates signalling via Toll-like receptor (TLR)-, NOD-like receptors (NLR)- and Formyl Peptide Receptor (FPR)- dependent pathways, as measured by kinase phosphorylation, NF-κB activation and cytokine production. TLR and FPR signalling pathways can be modulated by extracellular compounds. However, the ligands for NLRs need to be delivered intracellularly either by bacteria that invade the cell or through other cellular uptake mechanisms to be detected by for instance NOD1 and NOD2. This indicates that the short peptides are capable of exerting their effects via an intracellular mechanism. In example 4 the capacity of tetrapeptides, tripeptides and dipeptides to modulate TLR4 signalling, as measured by LPS induced PKB activation, is determined. Tetrapeptides AQGV and LQGV reduce LPS induced PKB activation. Surprisingly, dipeptides AQ, LQ and GV show a similar activity as the tetrapeptides AQGV and LQGV. Example 4 further demonstrates that the dipeptides are further capable of modulating both TLR4 mediated cell survival pathways, as shown by influencing LPS induced PKB/AKT phosphorylation and TLR4 mediated cytokine response pathways, as evidenced by influencing LPS induced P38 MAPK phosphorylation.

Without wishing to be bound by theory, it is thought that when a short peptide encounters a cell of the innate immune system (for example a monocyte or leukocyte or an intestinal epithelial cell), it is hydrolysed extracellularly at the surface of that cell. It is thought to be the activity of the dipeptide resulting in the observed activity. The short peptides of 3-12 amino acids serve as a so called delivery system for the dipeptides, and get hydrolysed into the active dipeptides at the cell surface.

Small peptides of two to six amino acids are known to serve as important nutritional sources of amino acids. Many extracellular peptidases are known to exist on innate immune cells that can rapidly hydrolyze peptides. Tripeptides, dipeptides and single amino acids will result from the hydrolysis of the short peptides. Such tripeptides, dipeptides and single amino acids can actively be transported through the cell membrane, whereby dipeptides are transported even faster than single amino acids because their uptake involves a separate mechanism. In mammals two transporters, PEPT1 and PEPT2, have been found to transport di- and tripeptides. The PEPT1 en PEPT2 transporters display a unique substrate specificity. They essentially transport all possible di- and tripeptides composed of L-α amino acids as well as a large variety of derivatives, such as peptides that contain D-enantiomers of amino acids (Cai H. et al. 2006, Genetics, 172:1459-1476 and Daniel, H. et al. 2006, Physiology, 21: 93-102). Thus until the present invention, uptake of di- and tripeptides into cells was known to be important for amino acid metabolism. However, the present invention shows for the first time that dipeptides consisting of L-amino acids have another function intracellularly as well, as modulators of signalling pathway, in particular Pattern recognition receptor (PRR) signalling pathways and G protein-coupled receptor (GPCR) signalling pathways.

The invention therefore provides a method for determining whether a peptide consisting of 2-12 amino acids is capable of modulating signalling via at least one Pattern Recognition Receptor (PRR) signalling pathway and/or G protein coupled receptor (GPCR) signalling pathway, the method comprising:
a) providing a cell susceptible to signalling through at least one of said pathways,
b) contacting said cell with at least one peptide,
c) contacting said cell with an agonistic or antagonistic compound of a PRR and/or a GPCR of said pathway, and
d) determining the activity of said at least one signalling pathway in said cell.

A method of the invention allows for selecting peptides which are capable of modulating at least one PRR signalling pathway and/or GPCR signalling pathway. In a preferred embodiment said at least one PRR signalling pathways is a Toll-like receptor (TLR) signalling pathway, preferably a TLR4 or TLR5 signalling pathway. Preferably, in a method of the invention a cell is contacted with an agonistic or antagonistic compound of a TLR, preferably TLR4 or TLR5.

In another preferred embodiment said at least one PRR signalling pathways is an NOD-like receptor (NLR) signalling pathway, preferably an NOD1 or NOD2 signalling pathway. Preferably, in a method of the invention a cell is contacted with an agonistic or antagonistic compound of an NLR signalling pathway, preferably an NOD1 or NOD2 signalling pathway.

In another preferred embodiment said at least one PRR signalling pathways is a Formyl Peptide Receptor (FPR) signalling pathway, preferably an FPR1, FPR2/ALX or FPR3 signalling pathway. Preferably, in a method of the invention a cell is contacted with an agonistic or antagonistic compound of an FPR signalling pathway, preferably an FPR1, FPR2/ALX or FPR3 signalling pathway.

In another preferred embodiment said at least one PRR signalling pathways is a retinoic acid inducible gene I (RIG-I)-like receptors (RLR) signalling pathway. Preferably, in a method of the invention a cell is contacted with an agonistic or antagonistic compound of an RLR signalling pathway.

In another preferred embodiment said at least one GPCR signalling pathways is a chemokine signalling pathway. Preferably, in a method of the invention a cell is contacted with an agonistic or antagonistic compound of a chemokine signalling pathway.

"A PRR or GPCR signalling pathway" refers to the transduction via an intracellular signalling molecule or cascade of signalling molecules in a cell following binding of an intracellular or extracellular ligand to a PRR or GPCR. The result of transduction of a signal or signalling cascade is typically a change in the expression of DNA in the nucleus or activation or inhibition of enzymes in the cytoplasm. As used herein "a PRR or GPCR signalling pathway" thus includes a Pattern recognition receptor or a G protein-coupled receptor and an intracellular signalling molecule or cascade of signalling molecules, but excludes a cellular response such as a change in the expression of DNA in the nucleus or activation or inhibition of enzymes in the cytoplasm.

"Modulating signalling via at least one PRR and/or GPCR signalling pathway" indicates that at least one PRR, such as a TLR4, an NOD, an FMLR, or one GPCR, such as an FPR or a chemokine signalling pathway is modulated. The modulation can be inhibition or potentiation. The result being that result of the signalling is lessened (inhibited) or heightened (potentiated), i.e. gene expression is less pronounced or more pronounced and/or activity of enzymes in the cytoplasm is reduced or increased.

In a preferred embodiment, it is determined whether a peptide is capable of modulating at least two PRR and/or GPCR signalling pathways. Modulation of two or more signalling pathways includes modulation of two or more different PRR signalling pathways, modulation of at least one PRR signalling pathway and at least one GPCR signalling pathway and modulation of two or more different GPCR signalling pathways. Modulation of two or more different PRR signalling pathways includes for instance at least one TLR- and at least one FPR- signalling pathway, or at least one NLR- and at least one GPCR-signalling pathway, or at least two different TLR signalling pathways, for instance TLR4 and TLR5 signalling. Said two or more signalling pathways can all be inhibited, or said two or more signalling pathways can all be potentiated. Alternatively, at least one of said signalling pathways is inhibited and at least one of said pathways is potentiated. For instance, a peptide can be identified with a method of the invention as an inhibitor of TLR4 signalling pathways and as a potentiator of an NOD signalling pathway.

Preferably, modulating signalling in a method of the invention is inhibiting said signalling or potentiating said signalling. A compound is an agonist or an antagonist of a PRR and/or a GPCR of a signalling pathway. In a specific embodiment, a compound is an agonist and said modulating signalling is inhibiting signalling.

In a preferred embodiment a peptide used in a method according to the invention is a dipeptide, preferably a dipeptide consisting of L-amino acid residues. More preferably, a peptide used in a method according to the invention is a dipeptide selected from the group of dipeptides having the general formula H-Xaa1-Xaa2-OH and wherein Xaa1 and Xaa2 are each independently selected from the group of amino acid residues A, C, D, E, F, G, H, I, K, L, M, N, P, Q, R, S, T, V, W and Y. More preferably, the amino acid residue at position Xaa1 and/or Xaa2 of said dipeptide is not Q.

In another preferred embodiment, a peptide used in a method according to the invention consists of 3-12 amino acids. It is preferred that the peptide tested in a method of the invention is smaller than 10 amino acids (aa), preferably smaller than 9 aa, more preferable smaller than 8 aa, most preferably smaller than 7 aa, even more preferably consisting of 3-6 aa, even more preferably consisting of 4-6 aa. Mixtures of peptides to be tested in a method of the invention preferably comprise at least one peptide that is smaller than 10 amino acids (aa), preferably smaller than 9 aa, more preferable smaller than 8 aa, most preferably smaller than 7 aa, even more preferably consisting of 3-6 aa, even more preferably consisting of 4-6 aa, preferably equal to 2 aa. It is preferred that all peptides in a mixture to be tested in a method according to the invention are smaller than 10 amino acids (aa), preferably smaller than 9 aa, more preferable smaller than 8 aa, most preferably smaller than 7 aa, even more preferably consisting of 3-6 aa, even more preferably consisting of 4-6 aa, preferably equal to 2 aa.

As used herein "peptide" include polymers of two or more amino acids. A peptide may be a "virtual" (or *in silico*) breakdown product of a larger native protein. As used herein, a "purified or isolated" peptide is a peptide that has been purified from a natural or biotechnological source or that has been synthesized. Any source comprising one or more peptides or salts or esters thereof, each of said peptides consisting of from two (2) to twelve (12) amino acids, can be used as starting material in a method of the invention. Preferably, a peptide comprises breakdown products of a naturally occurring protein, e.g. protein fragments (peptides) that are released during cell lysis or peptide hydrolysis or peptides which correspond with breakdown products but that have instead been synthesised according to routine peptide synthesis.

As used herein, a peptide or dipeptide includes variations made with regard to a reference peptide, wherein the variant retains an identifiable relationship to the reference peptide and retains the desired function of the reference peptide. Variants include, but are not limited to, compounds having the same or equivalent sidechains as the reference peptide arranged sequentially in the same order as the reference peptide, but for example, joined together by non-peptide bonds (*e.g*., by isosteric linkages such as the keto isostere, hydroxy isostere, diketo isostere, or the keto-difluoromethylene isostere), non-naturally occurring amino acids or polyamides, surrogate peptide bonds *(see, e.g.,* U.S. Patent 6,689,75, including, but are not limited to, CH₂, CH₂CH₂, CH=CH, C=C, CH₂NH, COCH₂, CH₂S, CH₂SO₂, and NHCO replacement of amid bonds), amidation of one or more carbon substitutions of an L-amino acid residue with a D-amino acid residue, and/or conjugated to a sugar, lipid, another polypeptide, nucleic acid and/or PNA. A functional analogue or derivative may be considered a peptide for the purposes of screening, identification of activity, inclusion in a database, production of a pharmaceutical and used in the method of identifying hit and lead compounds. In a preferred embodiment of the invention all amino acid residues of a peptide or dipeptide are L-amino acid residues.

The term "peptide" further includes a peptide which has been modified by, modifications such as, but not limited to, glycosylation, PEGylation, PEG alkylation, alkylation, acteylation, amidation, glycosyl-phophatdylinositalization, farnesylation, ADP-ribosylation, sulfation, lipid attachment, hydroxylation, and phosphorylation. Substitution of an amino acid other than those specifically exemplified or naturally present in a peptide of the invention are also contemplated within the scope of the present invention. For example, a non-natural amino acid can be substituted for an amino acid of a peptide, so long as the peptide having the substituted amino acid retains substantially the same functional activity as the peptide in which said amino acid has not been substituted. Examples of non-natural amino acids include, but are not limited to, ornithine, citrulline, hydroxyproline, homoserine, phenylglycine, taurine, iodotyrosine, 2,4-diaminobutyric acid, 4-aminobutyric acid, 2-amino butyric acid, 6-amino hexanoic acid, 2-amino isobutyric acid, 3-amino propionic acid, norleucine, norvaline, sarcosine, homocitrulline, cysteic acid, phenylglycine, cyclohexylalanine, fluoro-amino acids, designer amino acids such as C-methyl amino acids, N-methyl amino acids, and amino acid analogues in general. Non-natural amino acids also include amino acids having derivatized side groups. Furthermore, any of the amino acids in the protein can be of the D (dextrorotary) form, or of the L (levorotary) form. Allelic variants of a protein sequence of the present invention are also encompassed within the scope of the invention.

Considering the herein proposed mode-of-action of oligopeptides wherein hydrolysation of oligopeptides occurs at the cell surface into bioactive regulatory dipeptides that can then actively be transported into the cell by dipeptide transporters such as PEPT1 and PEPT2, a dipeptide tested in a method according to the invention is preferably limited to a set of 400 naturally occurring dipeptides composed of only L-amino acids according to the general formula H-Xaa1-Xaa2-OH, wherein Xaa1 and Xaa2 are each independently selected from any of the amino acids A, C, D, E, F, G, H, I, K, L, M, N, P, Q, R, S, T, V, W and Y, and wherein the dipeptides can be free bases or natural salts, such as acetate or HCl salts or easily dissolvable salts thereof. In certain embodiments, when one desires to rule out metabolic effects of dipeptides, it is also preferred that the amino acid at position Xaa1 and/or Xaa2 is not Q (Gln), N-terminal Q in dipeptides being easily susceptible to pyroglutamate formation, and C-terminal Q in dipeptides having known metabolic effects on cells, e.g. the Warburg effect. It is preferred that the dipeptides tested in a method as provided herein do not carry additional groups such as formyl, acetyl, muramyl, Fboc, or other reactive or protective groups added by microbial organisms or classical peptide chemistry and also is not desirable that said dipeptides are cyclized. A method of the invention thus preferably comprises contacting a cell with at least one dipeptide, wherein said dipeptide is selected from the group of 400 naturally occurring dipeptides having the general formula H-Xaa1-Xaa2-OH, wherein Xaa1 and Xaa2 are each independently selected from the group of L-amino acids A, C, D, E, F, G, H, I, K, L, M, N, P, Q, R, S, T, V, W and Y.

A dipeptide as described herein is preferably selected from AA, AC, AD, AE, AF, AG, AH, AI, AK, AL, AM, AN, AP, AQ, AR, AS, AT, AV, AW, AY, CA, CC, CD, CE, CF, CG, CH, CI, CK, CL, CM, CN, CP, CQ, CR, CS, CT, CV, CW, CY, DA, DC, DD, DE, DF, DG, DH, DI, DK, DL, DM, DN, DP, DQ, DR, DS, DT, DV, DW, DY, EA, EC, ED, EE, EF, EG, EH, EI, EK, EL, EM, EN, EP, EQ, ER, ES, ET, EV, EW, EY, FA, FC, FD, FE, FF, FG, FH, FI, FK, FL, FM, FN, FP, FQ, FR, FS, FT, FV, FW, FY, GA, GC, GD, GE, GF, GG, GH, GI, GK, GL, GM, GN, GP, GQ, GR, GS, GT, GV, GW, GY, HA, HC, HD, HE, HF, HG, HH, HI, HK, HL, HM, HN, HP, HQ, HR, HS, HT, HV, HW, HY, IA, IC, ID, IE, IF, IG, IH, II, IH, IL, IM, IN, IP, IQ, IR, IS, IT, IV, IW, IY, KA, KC, KD, KE, KF, KG, KH, KI, KK, KL, KM, KN, KP, KQ, KR, KS, KT, KV, KW, KY, LA, LC, LD, LE, LF, LG, LH, LI, LK, LL, LM, LN, LP, LQ, LR, LS, LT, LV, LW, LY, MA, MC, MD, ME, MF, MG, MH, MI, MK, ML, MM, MN, MP, MQ, MR, MS, MT, MV, MW, MY, NA, NC, ND, NE, NF, NG, NH, NI, NK, NL, NM, NN, NP, NQ, NR, NS, NT, NV, NW, NY, PA, PC, PD, PE, PF, PG, PH, PI, PK, PL, PM, PN, PP, PQ, PR, PS, PT, PV, PW, PY, QA, QC, QD, QE, QF, QG, QH, QI, QK, QL, QM, QN, QP, QQ, QR, QS, QT, QV, QW, QY, RA, RC, RD, RE, RF, RG, RH, RI, RK, RL, RM, RN, RP, RQ, RR, RS, RT, RV, RW, RY, SA, SC, SD, SE, SF, SG, SH, SI, SK, SL, SM, SN, SP, SQ, SR, SS, ST, SV, SW, SY, TA, TC, TD, TE, TF, TG, TH, TI, TK, TL, TM, TN, TP, TQ, TR, TS, TT, TV, TW, TY, VA, VC, VD, VE, VF, VG, VH, VI, VK, VL, VM, VN, VP, VQ, VR, VS, VT, VV, VW, VY, WA, WC, WD, WE, WF, WG, WH, WI, WK, WL, WM, WN, WP, WQ, WR, WS, WT, WV, WW, WY, YA, YC, YD, YE, YF, YG, YH, YI, YK, YL, YM, YN, YP, YQ, YR, YS, YT, YV, YW and YY, wherein the amino acids are indicated using the two-letter amino acid code. More preferably, a dipeptide is selected from AA, AG, AL, AP, AQ, AV, GA, GG, GL, GP, GQ, GV, LA, LG, LL, LP, LQ, LV, PA, PG, PL, PP, PQ, PV, QA, QG, QL, QP, QQ, QV, VA, VG, VL, VP, VQ, VV, MT, TR, RV, VL, LQ, QG, GV, LP, PA, AL and PQ. Even more preferably, a dipeptide is selected from AQ, MT, TR, RV, VL, LQ, QG, GV, LP, PA, AL and PQ. In a preferred embodiment a dipeptide is selected from AQ, LQ, GV and QG. In a more preferred embodiment a dipeptide is dipeptide GV. In another preferred embodiment, a dipeptide is selected that is capable of modulating signalling via at least one PRR signalling pathway and/or GPCR signalling pathway. A dipeptide preferably consists of L-amino acids and is selected from the group of 400 naturally occurring dipeptides having the general formula H-Xaa1-Xaa2-OH, wherein Xaa1 and Xaa2 are each independently selected from the group of L-amino acids A, C, D, E, F, G, H, I, K, L, M, N, P, Q, R, S, T, V, W and Y.

Where the herein described proteins, polypeptides, peptides or fragments thereof are said to be "human," this denotes that their primary sequence is the same as a corresponding primary sequence of, or present in, a naturally occurring human protein, polypeptide, peptide, or fragment thereof. Thus, the term "human" in this context relates to the primary sequence of the respective peptides, rather than to their origin or source. For example, a "human" peptide may be isolated from samples of human subjects, or may be obtained by other means (e.g., recombinant expression, cell-free translation, or non-biological peptide synthesis).

As used herein, "compound" and/or "peptide" includes an acceptable salt or ester of the compound or peptide. As understood in the art, an "acceptable salt or ester" refers to a salt or ester that retains the desired activity of the peptide or compound, and preferably does not detrimentally affect a subject, for example, a human subject. Preferably said acceptable salt or ester is a pharmaceutically acceptable salt or ester. Examples of such salts are acid addition salts formed with inorganic acids, for example, hydrochloric acid, hydrobromic acid, sulfuric acid, phosphoric acid, nitric acid, and the like. Salts may also be formed with organic acids such as, for example, acetic acid, oxalic acid, tartaric acid, succinic acid, maleic acid, fumaric acid, gluconic acid, citric acid, malic acid, ascorbic acid, benzoic acid, tannic acid, pamoic acid, alginic acid, polyglutamic acid, and the like. Salts may be formed with polyvalent metal cations such as zinc, calcium, bismuth, barium, magnesium, aluminum, copper, cobalt, nickel and the like or with an organic cation formed from N,N'-dibenzylethylenediamine or ethylenediamine, or combinations thereof (e.g., a zinc tannate salt).

A peptide used in a method according to the invention consisting of 3-12 amino acids is preferably composed of two alternating amino acids. More preferably said amino acids are selected from the group of L-amino acids A, C, D, E, F, G, H, I, K, L, M, N, P, Q, R, S, T, V, W and Y. More preferably said amino acid residue is not Q.

This way, dipeptides formed at the cell surface as a result of hydrolysis, are mainly identical dipeptides. Thus, peptides consisting of 3-12 amino acids used in a method of the invention serve as so called "delivery peptides" for dipeptides. For instance, a peptide consisting of 6 amino acids, composed of two different amino acids as building blocks, X₁ and X₂ (wherein X₁ and X₂ independently represent any amino acid), has the amino acid sequence X₁X₂X₁X₂X₁X₂. As another example a peptide consisting of 5 amino acids, composed of two different amino acids as building blocks, X₁ and X₂ (wherein X₁ and X₂ independently represent any amino acid), has the amino acid sequence X₁X₂X₁X₂X₁.

In a preferred embodiment a peptide with a length of 3-12 amino acid, which is composed of two different alternating amino acids as building blocks X₁ and X₂, is selected of which both possible dipeptides resulting from hydrolysis, i.e. dipeptide X₁X₂ and dipeptide X₂X₁, are capable of modulating signalling via at least one PRR signalling pathway and/or GPCR signalling pathway.

In another preferred embodiment, with a method according to the invention a dipeptide is selected consisting of an N-terminal amino acid X₁ and a C-terminal amino acid X₂ (i.e. dipeptide X₁X₂), which amino acids also form a dipeptide capable of modulating signalling via a PRR and/or a GPCR signalling pathway if X₂ is the N-terminal amino acid and X₁ is the C-terminal amino acid (i.e. dipeptide X₂X₁), wherein X₁ and X₂ independently represent any amino acid. For instance, dipeptide AL (alanine-leucine) is selected if dipeptide AL and LA (leucine-alanine) are both capable of modulating signalling via a PRR and/or a GPCR signalling pathway. Alternatively, dipeptides X₁X₂ and X₂X₁ are both selected with a method according to the invention.

Any cell susceptible to signalling through at least one PRR, such as TLR, NLR, FPR, RLR, and/or GCPR, such as chemokine-, signalling pathway can be used in a method of the invention. Preferably, a cell used in a method according to the invention is a mammalian cell, preferably a human cell. For example, it is a cell of a cell line which is easily maintained under laboratory conditions. As another example, the cell is a primary cell isolated from a subject. A preferred cell includes a cell typically involved in the innate immune system, for an instance antigen presenting cell (APC) such as a monocyte or a dendritic cell, preferably a PMBC-derived monocyte or dendritic cell, a macrophage, a granulocyte, an intestinal cell, a kidney cell, and another cell (such as CACO-2 cell) with documented TLR, NLR, RLR, FPR or GCPR expression.

A method of the invention preferably further comprises comparing said activity with a reference. One example of a reference is a cell susceptible to signalling through at least one PRR and/or GPCR signalling pathway which is contacted with an agonistic or antagonistic compound of a PRR and/or a GPCR of said pathway and which has not been contacted with a peptide. Another example of a reference is a cell susceptible to signalling through at least one PRR and/or GPCR signalling pathway which has been contacted with a peptide and which has not been contacted with an agonistic or antagonistic compound of a PRR and/or a GPCR of said pathway. Yet another example of a reference is a cell susceptible to signalling through at least one PRR and/or GPCR signalling pathway which has not been contacted with a peptide and which has not been contacted with an agonistic or antagonistic compound of a PRR and/or a GPCR of said pathway. Preferably a cell used in a method of the invention and a cell which is used as a reference are the same type of cell.

In a method of the invention, a cell can be pre-treated with at least one peptide consisting of 2-12 amino acids, followed by contacting said cell with at least one agonistic or antagonistic compound of a PRR or a GPCR of a signalling pathway. In a preferred embodiment, a cell is exposed to a compound selected from the group consisting of a TLR-2 ligand, a TLR-3 ligand, a TLR-4 ligand, a TLR-5 ligand, a flagellin, a peptidoglycan, an fMLP, and a combination thereof.

Peptides, preferably dipeptides, more preferably dipeptides consisting of L-amino acid residues, capable of modulating more than one distinct signalling pathway, i.e. having multiple targets, are of particular interest. Hence, in one aspect a method of the invention comprises determining whether a peptide is capable of modulating signalling via at least two PRR- (such as TLR, NLR, RLR and/or FPR) and/or GPCR (such as chemokine-) signalling pathways, and, if so, whether the peptide or peptide mixture does so in an agonistic, antagonistic, or mixed agonistic/antagonistic manner. For example, it involves determining whether a peptide or mixture of peptides are capable of modulating two or more signalling pathways selected from the group consisting of the TLR-2 signalling pathway, the TLR-3 signalling pathway, an NOD signalling pathway, the TLR-4 signalling pathway, the TLR-5 signalling pathway and an FPR signalling pathway. In a specific embodiment, it is determined whether a peptide is capable of modulating at least the TLR-4 induced signalling pathway and an FPR induced signalling pathway. In another specific embodiment, it is determined whether a peptide is capable of modulating at least the TLR-4 induced signalling pathway and the TLR-5 induced signalling pathway. In yet another specific embodiment it is determined whether a peptide are capable of modulating at least two different GPCR induced signalling pathways.

Preferred examples of an agonistic compound of TLRs include, but are not limited to, a TLR1-ligand (i.e lipoproteins, soluble lipoproteins, triacetylated lipopeptides are known to react with TLR1 complexed with TLR2); a TLR-2 ligand (i.e bacterial lipoproteins, lipoarabinomannan myccoplasma macrophage-activating lipopeptide 2 [MALP2] or MALP2 like substances such as disclosed in (WO/2006/138238) or derivatives of mycoplasma lipopeptide such as Clevland Biolabs substance CBLB 600 series [i.e substance CBLB612], glycolipids, porins); zymosan (known to react with TLR2 interacting with dectin); a TLR-3 ligand (i.e. double stranded DNA, polyinosine-polycytidylic acid [poly I:C], mRNA and tRNA); a TLR-4 ligand (i.e. lipopolysaccharides of S-or R-type, LPS-like substances, lipid-A, lipid-A-like subsyances, flavolipin, taxol-like substances, type III Repeat Extra Domain, hyaluronic acid, LMW oligosaccharides of hyaluronic acid, heparin sulphate, palyscahhraide fragments of heparin sulphate, fibrinogen and fragments thereof, fusion protein of respiratory syncytial virus, envelop protein of mouse mammary tumor virus glycoinositophospholipid); a TLR-5 ligand, (i.e, flagellin and flagellin-like substances such as disclosed in USPTO Patent Application 20090011982 or flagellin derivatives such as Cleveland Biolabs substance CBLB502 [Burdelya et al, Science Magazine April 2008, 320:226-230]); a TLR6 ligand (i.e. myccoplasma macrophage-activating lipopeptide 2 [MALP2]or MALP2 like substances such as disclosed in (WO/2006/138238) or derivatives of mycoplasma lipopeptide such as Clevland Biolabs substance CBLB 600 series [i.e substance CBLB612], diacylated lipoeptide or soluble tuberculosis factor); a TLR-7 ligand (i.e. single stranded RNA [ssRNA], imiquimod, gardiquimod, resiquimod, bropiriminem, U1snRNA, polyuridylic acid); a TLR 8 ligand (i.e. single stranded RNA [ssRNA]); a TLR-9 ligand (i.e. unmethylated CpG DNA, hemozoin); a TLR-11 ligand (i.e. profilin,); or other TLR-ligands known in the art. Other preferred examples of an agonistic compound include, but are not limited to, For-Met-Leu-Phe-OH [fMLP], Ethoxycarbonyl-Met-Leu-Phe-OMe, For-Met-Leu-Glu-OH, For-Ala-Ala-Ala-OH, For-Met-Lys-OH, For-Met-Ala-OH and other formyl-di-, tri-, tetrapeptides of bacterial or mitochondrial origin or synthesised version thereof known in the art, and any combination thereof and any combination of a TLR-ligand or an NLR ligand and any combination of an FPR-ligand and a TLR-ligand or any combination of an NLR-ligand and an FPR ligand. Preferred examples of an agonistic compound of a GPCR include, but are not limited to, GPCR-ligands such as adenosine, bradykinin, endothelin, γ-aminobutyric acid (GABA), hepatocyte growth factor (HGF), melanocortins, neuropeptide Y, opioid peptides, opsins, somatostatin, tachykinins, members of the vasoactive intestinal peptide family, vasopressin, dopamine, epinephrine, norepinephrine, histamine, glutamate, glucagon, acetylcholine, serotonin, chemokines; lipid mediators of inflammation (e.g., prostaglandins, prostanoids, platelet-activating factor, and leukotrienes); and peptide hormones (e.g., calcitonin, C5a anaphylatoxin, follicle-stimulating hormone (FSH), gonadotropin-releasing hormone (GnRH), neurokinin, thyrotropin-releasing hormone (TRH), and oxytocin) or any combination thereof, or any combination of a TLR-ligand, an NLR ligand, an FPR ligand, a RLR ligand and/or a GPCR ligand.

An effect of one or more peptides, preferably one or more dipeptides, on PRR-and/or GPCR signalling can be determined by methods known in the art based on a signalling event occurring downstream of the receptor. In a preferred embodiment, a method according to the invention comprises determining the activation state of at least one signalling molecule in said pathway. As used herein, "a signalling molecule" refers to any molecule in a PRR or GPCR signal transduction pathway, for instance in a PRR, TLR, NLR, FPR, RLR or GPCR signalling pathway, i.e. molecules involved in transduction of ligand binding to a PRR or GPCR to a cellular response. Such signalling molecules are located downstream of the receptor and include effector molecules such as for instance transcription factors.

Said activation state is preferably determined by measuring the phosphorylation status of one or more molecules involved in said PRR and/or GPCR signalling pathway. Such molecule involved in the signalling pathway is preferably a protein, preferably a cytosolic protein. The phosphorylation status may be determined relatively early after receptor modulation. For example, the phosphorylation status is determined within a time period of up to 1 hour after initiation of the PRR- and/or GPCR signalling pathway, like at 30 seconds, 60 seconds, 1, 5, 10, 15, 20, 30 and/or 60 minutes. Alternatively, or additionally, the phosphorylation status is determined relatively late after receptor modulation. Preferably, the phosphorylation status is determined within a time period of up from 1 to 24 hours after initiation of the PRR-and/or GPCR signalling pathway, like at 1.5, 2, 4 8, 10, 16 and/or 20 hr.

Measurement of the phosphorylation state of at least one molecule involved in said PRR and/or GPCR signalling pathway is an indicator of an interaction between a peptide and a PRR and/or GPCR signalling pathway. Greater phosphorylation of said at least one molecule indicates an agonistic interaction. Lesser phosphorylation of said at least one molecule indicates an antagonistic interaction.

In a preferred embodiment a signalling molecule is a TLR signalling molecule. Non-limiting examples of TLR signalling molecules include MAP3Ks; JNK; p38MAPK; PKB/Akt; NFkb; IkBa; IKKs; TRAF6; TAK1; IRAK1; IRAK4; TIRAP; TRAM; MyD88; TRIF; TBK1; IRF3; etc. Some TLR signalling molecules are known to be phosphorylated during TLR-mediated signalling; for example, PKB/Akt, JNK, p38MAPK, STAT3, and p42/p44MAPK. Measuring the phosphorylation state of these molecules, compared to control levels, allows for measurement of the extent of activation of TLR signal transduction in a cell.

A preferred signalling protein whose phosphorylation status can be indicative of PRR- (such as TLR-, NLR-, FPR-, RLR-) and/or GPCR signalling includes a protein kinase, preferably a tyrosine kinase, more preferably selected from the group consisting of PKB/Akt, P13K, JNK, p38MAPK and p42/p44MAPK. Another useful protein to evaluate includes another kinase, such as MSK, AMPK, GSK3, and a transcription factor, for example a STAT-family member, such as STAT3, or CREB.

Another preferred embodiment of the invention when studying for example innate immunity comprises measuring NF-κB activity. NF-κB activity is for instance measured by determining P50 and p65 NF-kB subunit activity by ELISA (for instance using ELISA of Cayman chemicals). This assay allows the determination of the true NF-kB activity by measuring the binding of its subunits to specific consensus sequences. For example, NF-κB activity is measured within a time period of up to 1 hour after initiation of the PRR-and/or GPCR signalling pathway, like at 30 seconds, 60 seconds, 1, 5, 10, 15, 20, 30 and/or 60 minutes. Alternatively, or additionally, NF-κB activity is measured relatively late after receptor modulation. Preferably, NF-κB activity is determined within a time period of up from 1 to 24 hours after initiation of the PRR- and/or GPCR signalling pathway, like at 1.5, 2, 4 8, 10, 16 and/or 20 hr.

Yet another preferred embodiment comprises measuring cytokine production, such as production of TNF-α, MIF, IL-1β, IL-6, IL-8, IL10, IL-12, IL-15, IL-18, IFN- α and/or IFN- β. Cytokine production is for instance measured by flow cytometry (for instance using Bender Flowcytomics kit, Bender Med systems, Burlingame, USA) or ELISA. Alternatively, cytokine production is measured by measuring gene expression of at least one gene encoding a cytokine. Preferably, cytokine production is determined within a time period of up from 0 to 72 hours after initiation of the PRR- and/or GPCR signalling pathway, like at 0.5, 1, 1.5, 2, 4 8, 10, 16, 20, 24, 36, 48, 60 and/or 72 hours.

Of course, the activity of a PRR and/or GPCR signalling pathway can also be determined by a combination of measuring the phosphorylation status of said at least one signalling molecule and/or measuring NF-κB activity and/or measuring production of inflammatory cytokines and/or any other method known in the art.

The skilled person will understand that various factors can influence the concentration at which a peptide, preferably a dipeptide, is used in a method according to the invention, such as cell type, nature of the peptide, signalling pathway under investigation, type of read-out used (e.g. protein kinase, transcription factor, cytokine production). Typically however, in a method according to the invention a cell is contacted with a peptide at a concentration of between about 1 and 1000 ng peptide/ ml, preferably between about 5 and 200 ng/ml, more preferably 10 and 100 ng/ml.

The present invention further provides peptides of 2-12 amino acids, particularly breakdown products of proteins, for use in therapeutic applications, preferably for therapeutic applications where modulation of (e.g., down-regulating or up-regulating) of at least one PRR, such as TLR, NLR, FPR or RLR, and/or GPCR, such as a chemokine, signalling pathway is desirable. A preferred embodiment provides a peptide, preferably a dipeptide, for use in modulating a PRR signalling pathway and/or a GPCR signalling pathway. Preferably, such peptides are selected using a method according to the invention. In a preferred embodiment a dipeptide consists of L-amino acid residues. In a more preferred embodiment a dipeptide is selected from the group of dipeptides having the general formula H-Xaa1-Xaa2-OH and wherein Xaa1 and Xaa2 are each independently selected from the group of amino acid residues A, C, D, E, F, G, H, I, K, L, M, N, P, Q, R, S, T, V, W and Y. In another preferred embodiment, the amino acid residue at position Xaa1 and/or Xaa2 of said dipeptide is not Q.

Other embodiments provide the use of at least one of such a small peptide in the preparation of a medicament for modulating, such as inhibiting or enhancing, at least one PRR and/or GPCR signalling. Provided is also a peptide consisting of 2-12 amino acids for use in modulating at least one PRR signalling pathway, such as a TLR, an FPR, an RLR, or a NLR pathway, and or a GPCR signalling pathway, such as a chemokine signalling pathway. In a preferred embodiment the invention provides a peptide consisting of 2-12 amino acids, preferably a dipeptide, for use in the treatment or prevention of infection with a pathogen or of sterile tissue injury caused by activation of the innate immune system with mitochondrial DAMPs. Preferably, a dipeptide consists of L-amino acid residues. More preferably, a dipeptide is selected from the group of dipeptides having the general formula H-Xaa1-Xaa2-OH and wherein Xaa1 and Xaa2 are each independently selected from the group of amino acid residues A, C, D, E, F, G, H, I, K, L, M, N, P, Q, R, S, T, V, W and Y. In another preferred embodiment, the amino acid residue at position Xaa1 and/or Xaa2 of said dipeptide is not Q.

In a preferred embodiment, peptide AQGV for use in inhibiting a TLR4 signalling pathway is provided. In another preferred embodiment, peptide LQGV for use in inhibiting a TLR4 signalling pathway is provided. In another preferred embodiment, peptide QGV for use in inhibiting a TLR4 signalling pathway is provided. In another preferred embodiment, peptide QGV for use in potentiating a TLR4 signalling pathway is provided. In another preferred embodiment, peptide AQG for use in potentiating a TLR4 signalling pathway is provided. In another preferred embodiment, peptide LQG for use in inhibiting a TLR4 signalling pathway is provided. In another preferred embodiment, peptide AQ for use in inhibiting a TLR4 signalling pathway is provided. In another preferred embodiment, peptide LQ for use in inhibiting a TLR4 signalling pathway is provided. In another preferred embodiment, peptide GV for use in inhibiting a TLR4 signalling pathway is provided. In another preferred embodiment, peptide GQ for use in potentiating a TLR4 signalling pathway is provided.

In another preferred embodiment, peptide AQGV for use in potentiating an NOD signalling pathway is provided. In another preferred embodiment, peptide AQGV for use in inhibiting an FPR signalling pathway is provided.

In another preferred embodiment, peptide AQGV for use in the treatment or prevention of infection with a pathogen or of sterile tissue injury caused by activation of the innate immune system with mitochondrial DAMPs is provided. In another preferred embodiment, peptide LQGV for use in the treatment or prevention of infection with a pathogen or of sterile tissue injury caused by activation of the innate immune system with mitochondrial DAMPs y is provided. In another preferred embodiment, peptide QGV for use in the treatment or prevention of infection with a pathogen or of sterile tissue injury caused by activation of the innate immune system with mitochondrial DAMPs is provided. In another preferred embodiment, peptide LQG for use in the treatment or prevention of infection with a pathogen or of sterile tissue injury caused by activation of the innate immune system with mitochondrial DAMPs is provided. In another preferred embodiment, peptide AQ for use in the treatment or prevention of infection with a pathogen or of sterile tissue injury caused by activation of the innate immune system with mitochondrial DAMPs is provided. In another preferred embodiment, peptide LQ for use in the treatment or prevention of infection with a pathogen or of sterile tissue injury caused by activation of the innate immune system with mitochondrial DAMPs is provided. In another preferred embodiment, peptide GV for use in the treatment or prevention of infection with a pathogen or of sterile tissue injury caused by activation of the innate immune system with mitochondrial DAMPs is provided. In another preferred embodiment, peptide QG for use in the treatment or prevention of infection with a pathogen or of sterile tissue injury caused by activation of the innate immune system with mitochondrial DAMPs is provided.

In another preferred embodiment, peptide LQ for use in prolonged treatment or prevention of infection with a pathogen or of sterile tissue injury caused by activation of the innate immune system with mitochondrial DAMPs is provided. In another preferred embodiment, peptide GV for use in prolonged treatment or prevention of infection with a pathogen or of sterile tissue injury caused by activation of the innate immune system with mitochondrial DAMPs is provided.

Also provided is a method for modulating a PRR signalling pathway and/or a GPCR signalling pathway, comprising administering a peptide consisting of 2-12 amino acids, preferably a dipeptide, to a cell susceptible to signalling through at least one of said pathways. In a preferred embodiment, a PRR signalling pathway and/or a GPCR signalling pathway is modulated in a subject, and the method comprises administering to said subject a therapeutically effective dose of a peptide consisting of 2 to 12 amino acids, preferably a dipeptide, more preferably a dipeptide consisting of L-amino acid residues. Also provided is a method for treating and/or preventing an infection with a pathogen or sterile tissue injury caused by activation of the innate immune system with mitochondrial DAMPs comprising administering a peptide consisting of 2-12 amino acids, preferably a dipeptide, more preferably a dipeptide consisting of L-amino acid residues, to a subject in need thereof. As used herein, the term "subject," or "patient," typically denotes a mammal, such as a human, a non-human primate, a rodent, a canine, a feline, an equine, an ovine, a porcine, etc. In a preferred embodiment a subject is a human. In another preferred embodiment, a dipeptide selected from the group of dipeptides having the general formula H-Xaa1-Xaa2-OH and wherein Xaa1 and Xaa2 are each independently selected from the group of amino acid residues A, C, D, E, F, G, H, I, K, L, M, N, P, Q, R, S, T, V, W and Y. In another preferred embodiment, the amino acid residue at position Xaa1 and/or Xaa2 of said dipeptide is not Q

A peptide used in a therapeutic application is preferably identified using a method according to the invention. Such a peptide is preferably a dipeptide, more preferably a dipeptide consisting of L-amino acid residues. A dipeptide used in a therapeutic application is preferably selected table 1. More preferably, said dipeptide is selected from table 2, even more preferably said dipeptide is selected from table 3, even more preferably said dipeptide is selected from table 4. In a preferred embodiment a dipeptide used in a method of the invention is thus selected from AQ, LQ, GV and QG. In a more preferred embodiment a dipeptide used in a method of the invention is GV. In another preferred embodiment, a dipeptide is selected that is capable of modulating signalling via at least one PRR signalling pathway and/or GPCR signalling pathway.

In a preferred embodiment, a method for modulating a PRR signalling pathway and/or a GPCR signalling pathway, comprises administering peptide AQGV to a cell susceptible to signalling through at least one of said pathways. In another preferred embodiment, a method for modulating a PRR signalling pathway and/or a GPCR signalling pathway, comprises administering peptide LQGV to a cell susceptible to signalling through at least one of said pathways. In another preferred embodiment, a method for modulating a PRR signalling pathway and/or a GPCR signalling pathway, comprises administering peptide QGV to a cell susceptible to signalling through at least one of said pathways. In another preferred embodiment, a method for modulating a PRR signalling pathway and/or a GPCR signalling pathway, comprises administering peptide LQG to a cell susceptible to signalling through at least one of said pathways. In another preferred embodiment, a method for modulating a PRR signalling pathway and/or a GPCR signalling pathway, comprises administering peptide AQ to a cell susceptible to signalling through at least one of said pathways. In another preferred embodiment, a method for modulating a PRR signalling pathway and/or a GPCR signalling pathway, comprises administering peptide LQ to a cell susceptible to signalling through at least one of said pathways. In another preferred embodiment, a method for modulating a PRR signalling pathway and/or a GPCR signalling pathway, comprises administering peptide GV to a cell susceptible to signalling through at least one of said pathways. In another preferred embodiment, a method for modulating a PRR signalling pathway and/or a GPCR signalling pathway, comprises administering peptide QG to a cell susceptible to signalling through at least one of said pathways.

In another preferred embodiment a method for treating and/or preventing an infection with a pathogen or sterile tissue injury caused by activation of the innate immune system with mitochondrial DAMPs comprises administering peptide AQGV to a subject in need thereof. In another preferred embodiment a method for treating and/or preventing an infection with a pathogen or sterile tissue injury caused by activation of the innate immune system with mitochondrial DAMPs comprises administering peptide LQGV to a subject in need thereof. In another preferred embodiment a method for treating and/or preventing an infection with a pathogen or sterile tissue injury caused by activation of the innate immune system with mitochondrial DAMPs comprises administering peptide QGV to a subject in need thereof. In another preferred embodiment a method for treating and/or preventing an infection with a pathogen or sterile tissue injury caused by activation of the innate immune system with mitochondrial DAMPs comprises administering peptide LQG to a subject in need thereof. In another preferred embodiment a method for treating and/or preventing an infection with a pathogen or sterile tissue injury caused by activation of the innate immune system with mitochondrial DAMPs comprises administering peptide AQ to a subject in need thereof. In another preferred embodiment a method for treating and/or preventing an infection with a pathogen or sterile tissue injury caused by activation of the innate immune system with mitochondrial DAMPs comprises administering peptide LQ to a subject in need thereof. In another preferred embodiment a method for treating and/or preventing an infection with a pathogen or sterile tissue injury caused by activation of the innate immune system with mitochondrial DAMPs comprises administering peptide GV to a subject in need thereof. In another preferred embodiment a method for treating and/or preventing an infection with a pathogen or sterile tissue injury caused by activation of the innate immune system with mitochondrial DAMPs comprises administering peptide QG to a subject in need thereof.

### Overview of several embodiments

Disclosed are methods for identifying a peptide having TLR-inhibitory activity, TLR-potentiating activity, NLR-inhibitory activity NLR-potentiating activity, FPR-inhibitory activity FPR-potentiating activity, RLR-inhibitory activity, RLR-potentiating activity, GPCR-inhibitory activity and/or GPCR-potentiating activity. For instance, methods of the invention involve contacting a cell expressing a TLR with a candidate peptide, contacting the cell with a TLR ligand, and assaying the cell for evidence of phosphorylation of downstream effectors of TLR-mediated signalling. In these and other embodiments, contacting a cell with a candidate peptide with TLR-inhibitory activity and a TLR ligand will lead to reduced phosphorylation of downstream effectors of TLR-mediated signalling (*e.g*., PKB/Akt; JNK; p38MAPK; p42/p44MAPK; etc.), relative to the amount of phosphorylation observed when the cell is contacted with a TLR ligand alone. In another example, methods of the invention involve contacting a cell expressing a NLR with a candidate peptide, contacting the cell with a NLR ligand, such as peptidoglycan, and assaying the cell for evidence of phosphorylation of downstream effectors of TLR-mediated signalling. In these and other embodiments, contacting a cell with a candidate peptide with NLR-potentiating activity and a NLR ligand will lead to increased phosphorylation of downstream effectors of NLR-mediated signalling (*e.g.*, PKB/Akt; JNK; p38MAPK; p42/p44MAPK; etc.), relative to the amount of phosphorylation observed when the cell is contacted with a NLR ligand alone. Further embodiments provide methods for identifying a small peptide with PRR and/or GPCR-inhibiting or PRR and/or GPCR -stimulatory activity. In certain embodiments, the small peptide, AQGV, is used to inhibit TLR4-induced signalling. In other embodiments, AQGV, a close variant of AQGV, is used to enhance NOD-induced signalling. In still further embodiments, the pentapeptide VLPALP is used to inhibit TLR4-induced signalling.

In particular embodiments, the TLR that is inhibited by a peptide of the invention is TLR4. In other embodiments, the TLR is, for example, TLR1; TLR2; TLR3; TLR4; TLR5; TLR6; TLR7; TLR8; TLR9; TLR10; TLR11; TLR12; or TLR13. In some embodiments, the TLR-peptide is, for example, a peptide derived from the hCG breakdown product, MTRVLQGVLPALPQVVC. In particular embodiments, the TLR-inhibitory peptide is AQGV. In other embodiments, the TLR-inhibitory peptide is, for example, LQGV or QGVA, AGVQ, VGQA, GVQA or LEGV, GVGV and AGAG or LSGV or AQG or LQG or QGV or AQ or LQ or QG or GV.

In some embodiments, the test peptide is, for example, an opioid peptide such as LVVYPWT, or a dimeric or trimeric or tetrameric or pentameric peptide derived from breakdown of LVVYPWT. In particular embodiments, the test peptide is YPWT or YP or PW or WT. In other embodiments, the test peptide is, for example, an opioid peptide YPWTQRF; or a dimeric or trimeric or tetrameric or pentameric peptide derived from breakdown of YPWTQRF, or YVPFPPF or YVPF or YPWF or YPFF or LTRPRY (see also EFSA Scientific Report (2009) 231, 14-107 for further examples).

### Methods of identifying modulators of PRR or GPCR signalling

Described herein is a general strategy for identifying peptides that inhibit or potentiate TLR, FPR, RLR, NLR, and/or GPCR signalling pathways which peptides may act as modulators of innate immunity and/or inflammatory signalling. As used herein, the term, "innate immunity" and similar phrases, are defined as the portion of the immune system comprising the cells and mechanisms that defend the host from infection by other organisms, in an antigen-non-specific, non-antibody-mediated manner. This means that the cells of the innate system (*e.g*., dendritic cells, macrophages, mast cells, neutrophils, etc.) recognize and respond to pathogens in a generic way, and, unlike the adaptive immune system (comprising the humoral and adaptive immune responses), it does not confer long-lasting or protective immunity to the host. Innate immune systems provide immediate defence against infection and are responsible for, for example, the inflammatory response. Inhibitory- or potentiating peptides identified by a method of the invention may further be useful in any application wherein inhibition or potentiating of said signalling pathway is desired. For instance, inhibiting of TLRs is desired for inhibiting growth of tumours comprising mutations in Kras and/or p53.

In some embodiments, a method of identifying modulators of innate immunity and inflammatory signalling comprises screening for a peptide's usefulness in the modulation of innate immunity. Screening for a peptide's usefulness as a modulator of innate immunity may be accomplished, for example, by providing a plurality of peptides or salts or esters thereof, contacting peptides thereof with for instance a TLR, and screening said peptides in order to identify peptides which interact with said TLR. Additionally, peptides identified in such screen as TLR-contacting peptides may be further recognized as peptides useful in TLR modulation, by determining whether they affect TLR-mediated signalling in an agonistic, antagonistic, or mixed agonistic/antagonistic manner. In some embodiments, peptides included within the plurality of peptides are from 2-12 amino acids in length. For example, peptides included within the plurality of peptides may be 2; 3; 4; 5; 6; 7; 8; 9; 10; 11; or 12 amino acids in length. In further embodiments, peptides included within the plurality of peptides may be more than 5, or more than 10, amino acids in length.

In particular embodiments, a Pattern Recognition Receptor (PRR) or G Protein-Coupled Receptor (GPCR) contacted with peptides to screen said peptides for PRR or GPCR signalling pathway modulatory function may be expressed on the surface of a cell. A cell expressing a PRR or a GPCR may be a cell wherein the PRR or GPCR is natively expressed, for example, a PBMC, or the cell may be one wherein the PRR or GPCR is heterologously expressed, *e.g*., by recombinant genetic techniques. In alternative embodiments, the PRR or GPCR contacted with peptides to screen said peptides for PRR or GPCR modulatory function may be located in a cell-free system, for example, an *in vitro* binding assay.

Interaction of a peptide with a PRR or GPCR signalling pathway may be determined by any method known to those of skill in the art. As used herein, the term "interact" refers to the quality, state or process of two or more elements, *e.g*., proteins or genes, acting on each other. Two or more proteins may interact, for example, by specific binding. Two or more proteins may also interact, for example, through intervening signalling molecules that transmit and/or amplify the receipt of a stimulus between the two proteins. One or more genes may interact, for example, by contributing to a phenotype, such that mutations or changes in expression of one gene affects the contribution of the interacting genes to the phenotype. Thus, in general, elements "interact" if they share information within a system for affecting one or more properties; such as a cellular signal transduction pathway, or the expression of a phenotype in an organism. In some embodiments, an interaction between a peptide and a PRR or GPCR signalling pathway may be determined by identification of specific binding between the peptide and the PRR or GPCR. In other embodiments, an interaction may be determined by measuring activation, or inhibition of activation, of molecules involved in PRR- or GPCR-mediated signalling. For instance, physiological molecular signalling mechanisms have been partially described for all thirteen known TLRs, including molecules involved in such signalling. Krishnan et al. (2007) Exp. Mol. Med. 38:421-38; Roach et al. (2005) Proc. Natl. Acad. Sci. USA 102:9577-82; Janeway and Medzhitov (2002) Annu. Rev. Immunol. 20:197-216; Heil et al. (2004) Science 303:1526-9; Nishiya and De Franco (2004) J. Biol. Chem. 279:19008-17. For example, PKB/Akt, JNK, and p38MAPK, are phosphorylated following ligand binding to certain TLRs as a consequence of TLR-mediated signal transduction. Thus, measurement of the phosphorylation state of PKB/Akt, JNK, and/or p38MAPK in a cell expressing certain TLRs (*e.g*., TLR4) is an indicator of an interaction between a peptide and a TLR signalling pathway. Greater phosphorylation of these molecules exhibited in TLR-expressing cells contacted with a peptide indicates an agonistic interaction. Lesser phosphorylation indicates an antagonistic interaction. Peptides that interact with a TLR signalling pathway in an agonistic, antagonistic, or mixed agonistic/antagonistic manner, are modulators of TLR-mediated signalling, and thus, are modulators of innate immunity and inflammatory signalling.

In some embodiments, interaction of a peptide with a PRR or GPCR signalling pathway may be determined by measuring NF-κB activity. NF-κB is a major transcription factor that regulates genes responsible for both the innate and adaptive immune response. Stimulation of for instance TLRs (for example, stimulation of TLR4 with LPS) leads to increased NF-κB activity. Activation of NF-kappaB leads to translocation of NF-kappaB to the nucleus of the cell receiving the activating stimulus. NF-kappaB activity may be measured by any technique known in the art, for example, electrophoretic mobility shift assay (EMSA); immunofluorescence; and use of a reporter gene, such as luciferase, wherein the reporter gene is operably linked to an NF-κB responsive element.

### Modulators of innate immunity and inflammatory signalling

Any plurality of peptides (such as a plurality comprising randomly selected peptides, preferably dipeptides) may be screened according to methods of the invention to identify modulators of innate immunity and inflammatory signalling. However, those of skill in the art may choose one or a plurality of peptides by recognizing the plurality of peptides as likely to comprise modulators of innate immunity and inflammatory signalling, *e.g*., TLR-interacting peptides.

Without being bound by any specific theory, the inventors hypothesize that a signal exists to "turn off" pro-inflammatory responses mediated by PPRs. Strong inflammatory responses are induced by PAMPs. Since many of these PAMPs are proteins that are released from cells after lysis, it is proposed that the breakdown product peptides of proteins provide a signal to turn off TLR-mediated pro-inflammatory responses. Under this theory, when the level of the breakdown product peptides exceeds the level of the PAMP proteins, the inflammatory response is actively turned off. This hypothesis predicts that mixtures of proteolytic peptides may inhibit PPR activation, such as TLR activation. Also, breakdown product peptides that retain structure exhibited by the whole PAMP that is recognized by a PRR, such as a TLR, may act to stimulate pro-inflammatory responses. Thus, in some embodiments, a plurality of peptides is selected for screening, wherein the plurality of peptides comprises breakdown products of PAMP proteins released after cell lysis. In particular embodiments, the peptides may be breakdown products of hCG, of haemoglobin or of fibrinogen.

### Methods of modulating innate immunity and inflammatory signalling

Described herein are methods for the treatment of certain diseases or conditions, or predispositions thereto, in subjects. Peptides identified as modulators of PRR and/or GPCR signalling pathways may be administered to a subject, *e.g*., in order to increase or decrease innate immune responsiveness or inflammation. Thus, in some embodiments, the invention provides a pharmaceutical composition comprising an active component that is a peptide modulator of innate immunity and inflammatory signalling. Therefore, modulators of PRR and/or GPCR signalling pathways may be used for the treatment and prevention of a wide range of infectious diseases, for example, caused by fungi, bacteria, or viruses; in oncologic pathology (*e.g.*, treatment of Lewis lung carcinoma); for control of the innate immune response component of allergic or inflammatory diseases; and for enhancement of the action of vaccines when used wholly or partly as an adjuvant, etc.

Peptide modulators of PRR and/or GPCR signalling pathways of the invention may be administered to a subject by any method known in the art. In general, a pharmaceutical composition comprising one or more peptides identified as a modulator of PRR and/or GPCR signalling pathways will be administered to a subject, for example, parenterally; orally (in the case of small peptides only); transdermally; etc. Pharmaceutical compositions comprising one or more peptide modulators of PRR and/or GPCR signalling pathways of the invention may further comprise one or more pharmaceutically acceptable carriers and/or excipients.

Multiple proteolytic peptides that are modulators of PRR and/or GPCR signalling pathways, such as dipeptides, may be contained within a single polypeptide, such that when the polypeptide is administered to a subject, it is broken down to the individual active peptides. Thus, in some embodiments, the invention provides a pharmaceutical composition comprising one or more peptides identified as modulators of PRR and/or GPCR signalling pathways. In some embodiments, the invention provides a polypeptide that is broken down into one or more peptides identified as modulators of PRR and/or GPCR signalling pathways. Active peptides and polypeptides that are broken down to active peptides may be provided in the same composition.

In some embodiments, PRR and/or GPCR signalling may be modulated in a model system, for example, to facilitate the study of innate immune and inflammatory responses. In certain embodiments, a knockout mouse is generated using an identified sequence of a native peptide modulator of innate immunity and inflammatory signalling. For example, the mouse genome may be searched for nucleotide sequences that encode an identified peptide modulator of innate immunity and inflammatory signalling. Once identified and located in the mouse genome, the encoding sequence may be deleted or substituted from the mouse germline by techniques known to those of skill in the art. See, *e.g*., Waldman (1992) Crit. Rev. Oncol. Hematol. 12(1):49-64; Capecchi (2005) Nat. Rev. Genet. 6(6):507-12. Reproduction of the resultant "knockout mouse" will preserve the mutation such that progeny mice are incapable of expressing the peptide modulator of innate immunity and inflammatory signalling. In some embodiments, a "conditional knockout mouse" may be made, wherein the mouse does not express the peptide modulator of innate immunity and inflammatory signalling in a particular tissue or cell type, at a particular stage of development, or when the mouse is exposed to a stimulus provided by the researcher. The production of conditional knockout mice is also known in the art. See, *e.g*., Lobe and Nagy (1998) Bioessays 20(3):200-8; Bockamp et al. (2002) Physiol. Genomics 11(3):115-32.

The following Examples are provided to illustrate certain particular features and/or embodiments. These Examples should not be construed to limit the disclosure to the particular features or embodiments described.

### Figure legends

Figure 1. P50 and p65 NF-kB subunit activity in PBMC derived neutrophils stimulated with1µg/mL LPS or 1µg/mL LPS and 20 or 50 ng/mL of EA-230 (peptide AQGV).
Figure 2. A. TNFα production in PBMC derived neutrophils stimulated with 1 µg/mL LPS or 1 µg/mL LPS and 20 or 50 ng/mL of EA-230 (peptide AQGV). B. IL-6 production in PBMC derived neutrophils stimulated with 1 µg/mL LPS or 1 µg/mL LPS and 20 or 50 ng/mL of EA-230 (peptide AQGV). C. IL-8 production in PBMC derived neutrophils stimulated with 1 µg/mL LPS or 1 µg/mL LPS and 20 or 50 ng/mL of EA-230 (peptide AQGV). D. IL-10 production in PBMC derived neutrophils stimulated with 1 µg/mL LPS or 1 µg/mL LPS and 20 or 50 ng/mL of EA-230 (peptide AQGV).
Figure 3. β-actin normalized PKB (A) JNK (B), p38MAPK (C), and P42/p44MAPK/ERK1,2 (D) phosphorylation in PBMC derived monocytes following stimulation with 2 µg/mL LPS or stimulation with 2 µg/mL LPS and 20 or 50 ng/mL peptide AQGV (Biotempt peptide).
Figure 4. β-actin normalized PKB (A) JNK (B), p38MAPK (C), and P42/p44MAPK/ERK1,2 (D) phosphorylation in PBMC derived monocytes following stimulation with 2 µg/ml NOD ligand peptidodoglycan (PGN) or stimulation with 2 µg/ml PGN and peptide AQGV (Biotempt peptide).
Figure 5. β-actin normalized PKB (A) JNK (B), p38MAPK (C), and P42/p44MAPK/ERK1,2 (D) phosphorylation in PBMC derived monocytes following stimulation with 1 µM fmlp (formyl-Met-Leu-Phe) or stimulation with 1 µM fmlp and peptide AQGV (Biotempt peptide).
Figure 6. β-actin normalized PKB phosphorylation in PBMCs following stimulation with 2 µg/mL LPS (0 ng) or stimulation with 2 µg/mL LPS and 20 ng/mL of peptides AQGV, LQGV, AQG, LQG, QGV, AQ, LQ, GV or QG (20 ng).
Figure 7. Significant upregulation ("up") and significant downregulation ("down") of PKB phosphorylation in PBMCs following stimulation with 2 µg/mL LPS (0 ng) or stimulation with 2 µg/mL LPS and 1.3, 3.2, 8, 20 and 50 ng/mL of peptides AQGV, LQGV, AQG, LQG, QGV, AQ, LQ, GV or QG.
Figure 8. β-actin normalized p38 MAPK phosphorylation in PBMCs following stimulation with 2 µg/mL LPS (0 ng) or stimulation with 2 µg/mL LPS and 20 ng/mL of peptides AQGV, LQGV, AQG, LQG, QGV, AQ, LQ, GV or QG (20 ng).
Figure 9. Significant upregulation ("up") and significant downregulation ("down") of p38 MAPK phosphorylation in PBMCs following stimulation with 2 µg/mL LPS (0 ng) or stimulation with 2 µg/mL LPS and 1.3, 3.2, 8, 20 and 50 ng/mL of peptides AQGV, LQGV, AQG, LQG, QGV, AQ, LQ, GV or QG.

### Examples

### Example 1

### Material and methods

### Cell Isolation

Blood was drawn from healthy volunteers into tubes containing citrate as an anticoagulant. Neutrophils were isolated by using a Polymorphprep kit (Nicomed, Oslo, Norway) according to the manufacturer's instructions; monocytes were purified with magnetic beads (Miltenyi Biotech). The purity of the cells, as assessed by flow cytometry (anti-CD45, 14, DR, and CD66b), was > 93%. For each cell type, samples from two different donors were examined.

### Cell Stimulation and Protein extraction

5x10⁶ cells were incubated in the presence of 1 µg/mL LPS (*E.coli* O111:B4, Sigma-Aldrich) or pre-stimulated with 20 ng/mL of EA-230 (peptide AQGV) and then incubated in the presence of 1 µg/mL LPS for 0, 5, 15 and 30 minutes (37°C, 5% CO₂). At the end of the incubation period, the cells were put on ice, and proteins were extracted using Phosphosafe reagent (Novagen), according to the recommendations of the manufacturer. The total protein concentration was measured using a conventional Lowry assay (Pierce).

### Phosphoprotein array

To determine the relative levels of protein phosphorylation, a human PhosphoKinase array (RnD Systems) was used according to the recommendations of the manufacturer. 200 µg of protein were used per array. Spot pixel density was determined for each protein and ratios to the controls (no LPS and no EA stimulation) were measured.

### Results

### Neutrophils

In neutrophils, LPS treatment resulted in the phosphorylation of many proteins, and EA-230 blocked this effect. Specifically, at 15 minutes, EA-230 blunted the LPS-driven phosphorylation of P38 MAPK, ERK1/2, GSK 3a/b, MEK1/2 and CREB. EA-230 caused an increase in eNOS phosphorylation.

### Monocytes

EA-230 inhibits LPS-driven phosphorylation of proteins in monocytes. At 15 minutes, phosphorylation of a number of proteins was increased in the presence of LPS. Pre-incubation with EA-230 prevented this effect in the cases of P38 MAPK, ERK1/2, JNK, MSK, AMPK and PKB/AKT. In contrast, EA-230 strongly stimulated eNOS phosphorylation.

### Example 2

### Material and methods

### Cell Isolation

Blood was drawn from 2 healthy volunteers into tubes containing citrate as an anticoagulant. Neutrophils were isolated by using a Polymorphprep kit (Nicomed, Oslo, Norway) according to the manufacturer's instructions. The purity of the cells, as assessed by flow cytometry (anti-CD45, 14, DR, and CD66b), was > 93%.

### Cell Stimulation and Nuclear Protein extraction

5x10⁶ cells were incubated in the presence of 1 µg/mL LPS (*E.coli* O111:B4, Sigma-Aldrich) or pre-stimulated with 20 or 50 ng/mL of EA-230 (peptide AQGV) and then incubated in the presence of 1 µg/mL for 15 minutes (37°C, 5% CO₂). At the end of the incubation period, the cells were put on ice, and nuclear proteins were extracted using Nuclear Extraction Kit (Cayman chemicals, Ann Harbor, USA), according to the recommendations of the manufacturer. The total protein concentration was measured using a conventional Lowry assay (Pierce).

### NF-kB Activity

P50 and p65 NF-kB subunit activity was determined by ELISA (Cayman chemicals). This assay allows the determination of the true NF-kB activity by measuring the binding of its subunits to specific consensus sequences. This assay is similar to the traditional EMSA, though more sensitive. 10 µg of proteins were used per assay. Analysis was performed in duplicate.

### Cell Stimulation and cytokines measurement

5x10⁵ cells were incubated in the presence of 1 µg/mL LPS (*E.coli* O111:B4, Sigma-Aldrich) or pre-stimulated with 20 or 50 ng/mL of EA-230 (peptide AQGV) and then incubated in the presence of 1 µg/mL LPS for 24 hours (37°C, 5% CO₂). At the end of the incubation period, supernatants were harvested and concentrations of TNF-α, IL-6, and IL-8 were measured by flow cytometry using Bender Flowcytomics kit (Bender Med systems, Burlingame, USA) according to the recommendations of the manufacturer. Concentrations of IL-10 were determined by ELISA (RnD Systems). Measurements were done in duplicate.

### Statistics

Non parametric *U* test was used. Experiments were performed twice.

### Results

### EA-230 reduced the LPS-driven NF-kB activation

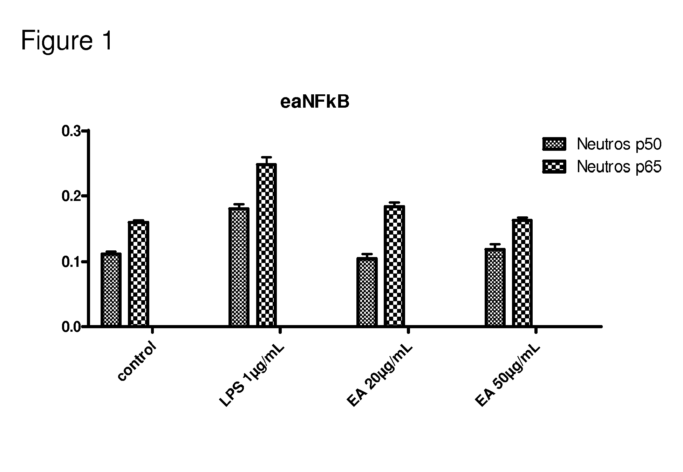

LPS induced NF-kB p50 and p65 activity as compared to unstimulated (control) cells (figure 1, p<0.02). EA-230 reduced this LPS induced activation at both concentrations (20 and 50 µg/mL) (p=0.03 vs LPS alone). There was no difference between the 2 EA-230 concentrations.

### EA-230 reduced the LPS-driven cytokine production

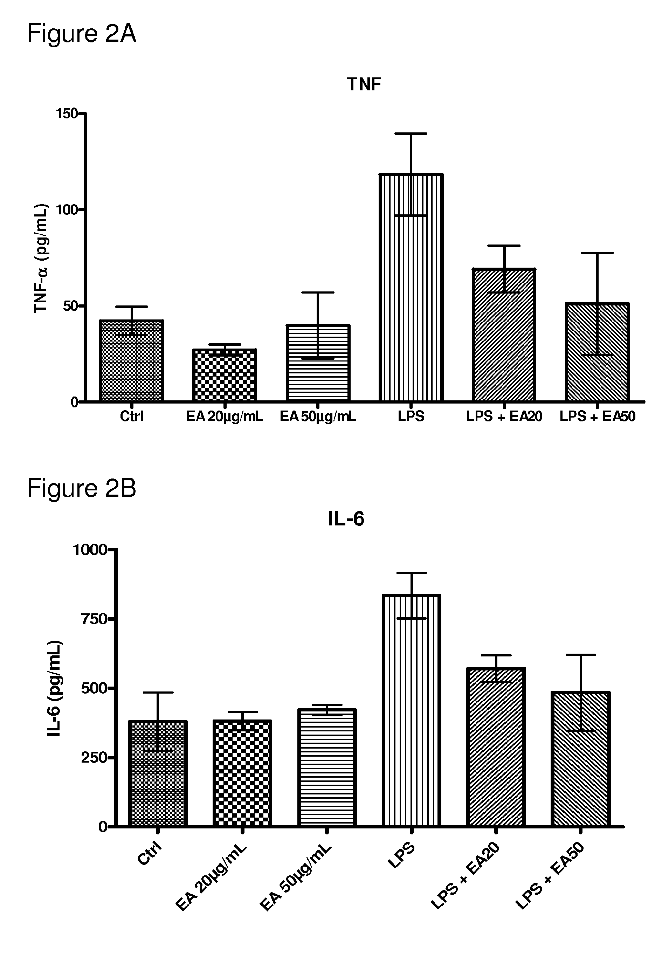

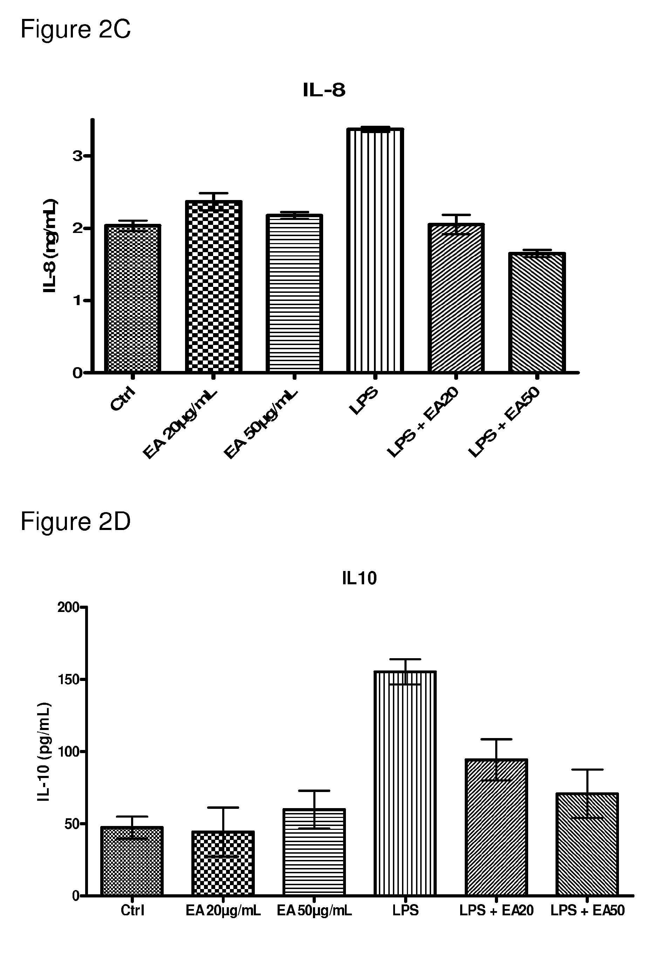

LPS induced the production of TNF-α, IL-6, IL-8 and IL-10 by neutrophils (figure 2, p<0.005 vs unstimulated cells). At both concentrations, EA-230 reduced the cytokine production almost to control values (p=0.01 versus LPS).

### Example 3

### Materials and methods

PMBC-derived monocytes were isolated from healthy volunteers (hPBMCs) according to routine procedures¹. Subsequently PMBC were incubated with 20 ng/ml peptide AQGV (Biotempt peptide, which was freshly prepared as a 20 mg/ml stock solution in bidistilled water) or treated with the cell culture medium alone for 10 min. hPMBC were challenged with:
1) 2 µg/ml TLR-4 ligand LPS ² for 1 min, 5 min, 10 min and 20 min,
2) 2 µg/ml NOD ligand peptidoglycan (PGN) for 1 min, 5 min, 10 min and 20 min, or
3) 1 µM fmlp (formyl-Met-Leu-Phe) for 1 min, 5 min, 10 min and 20 min.

Following stimulation, the phosphorylation status of p38MAPK, JNK, PKB, and P42/p44MAPK/ERK1,2 was analysed³. Results were corrected for differences using co-measurement of β-actin⁴.

### Results

The results show that peptide EA-230 (AQGV) is a potent and statistically significant immunomodulator.

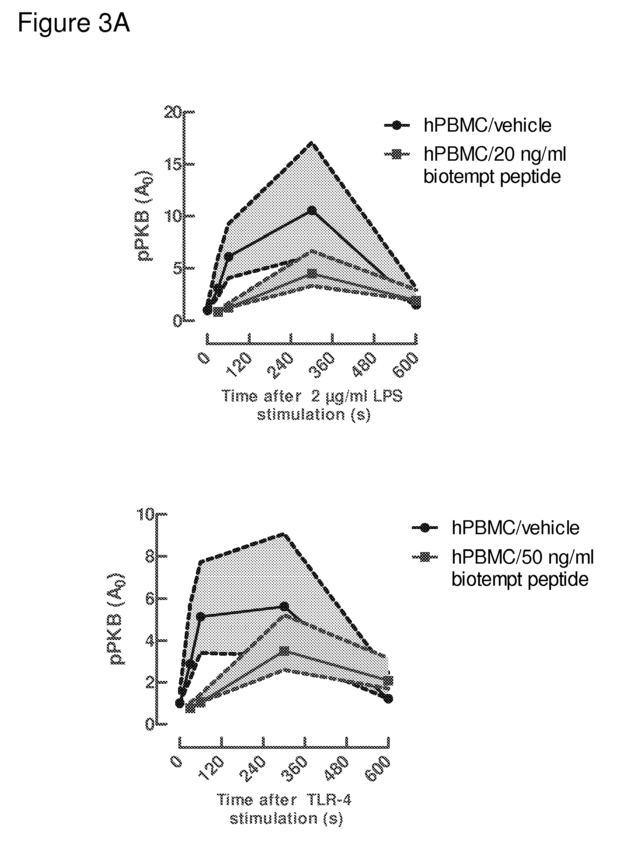

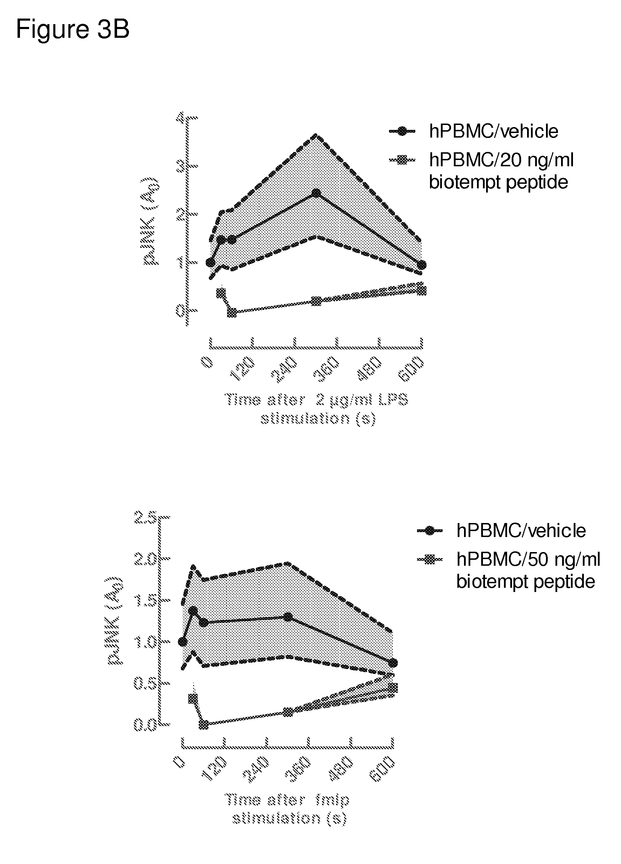

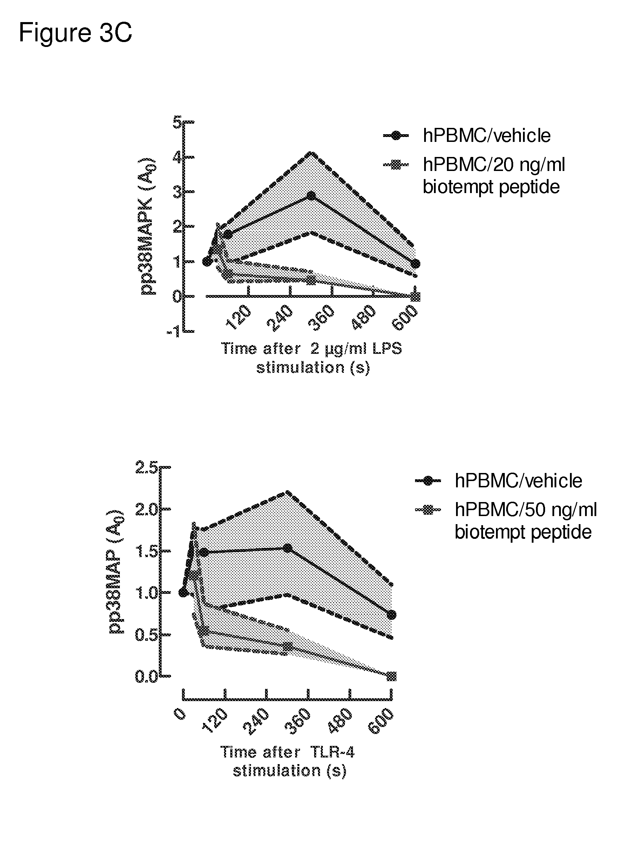

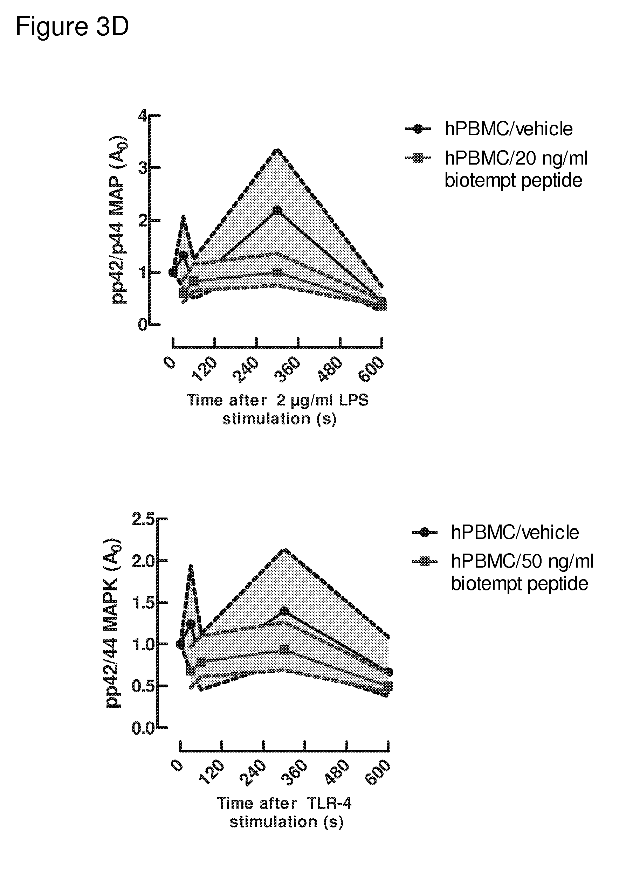

EA-230, upon acute incubation, reduced TLR-4 mediated signalling induced by LPS, as measured by p38MAPK, JNK, PKB, and P42/p44MAPK/ERK1,2 phosphorylation status, when tested at 20 ng/ml and at 50 ng/ml (figure 3).

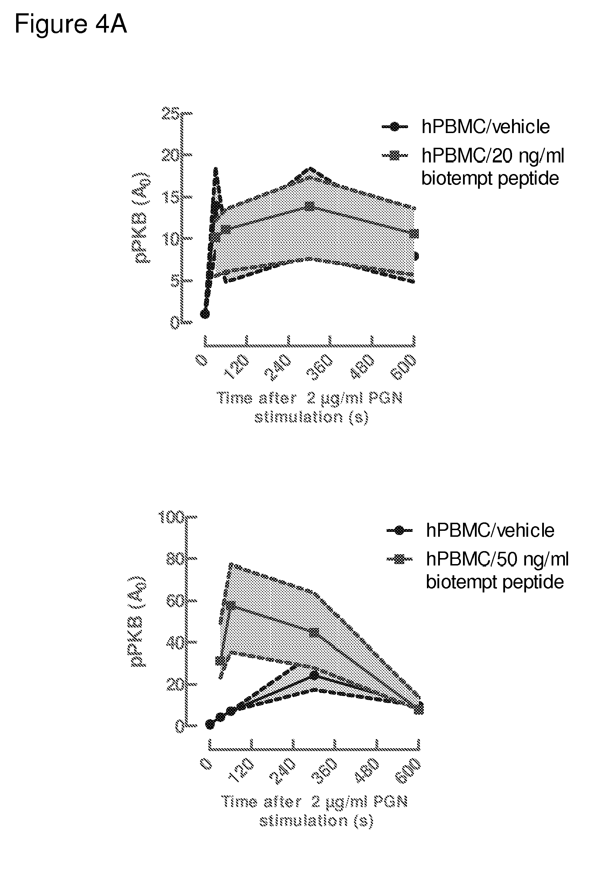

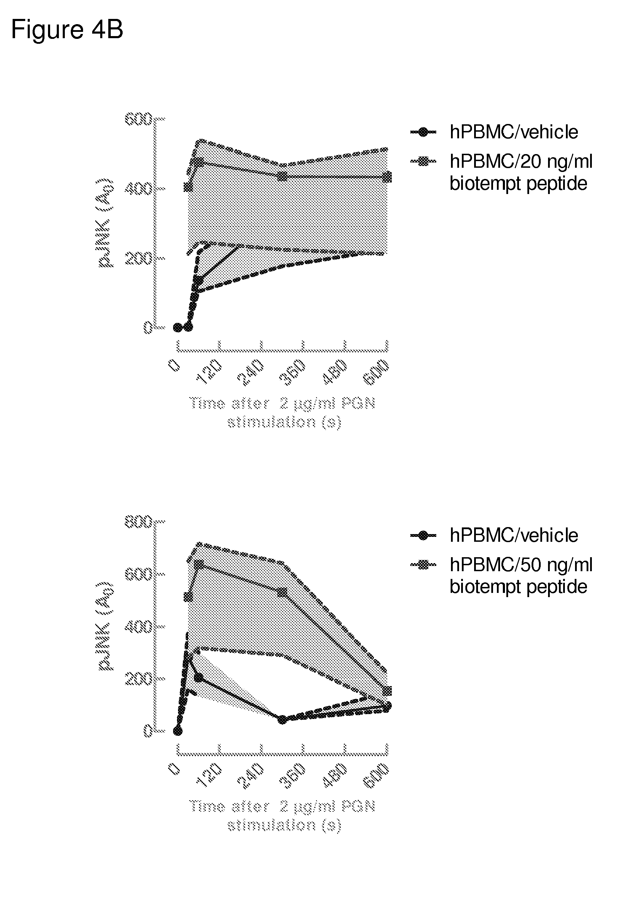

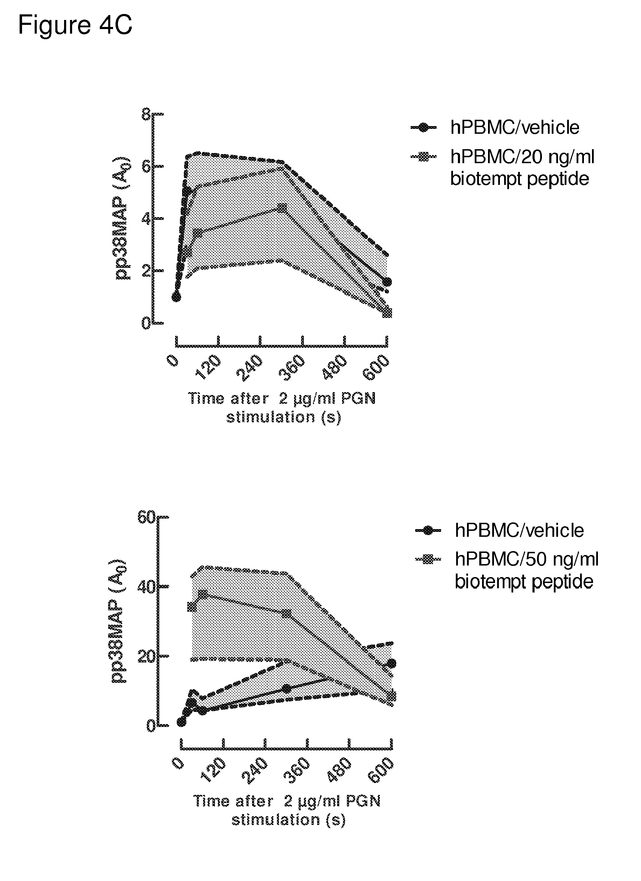

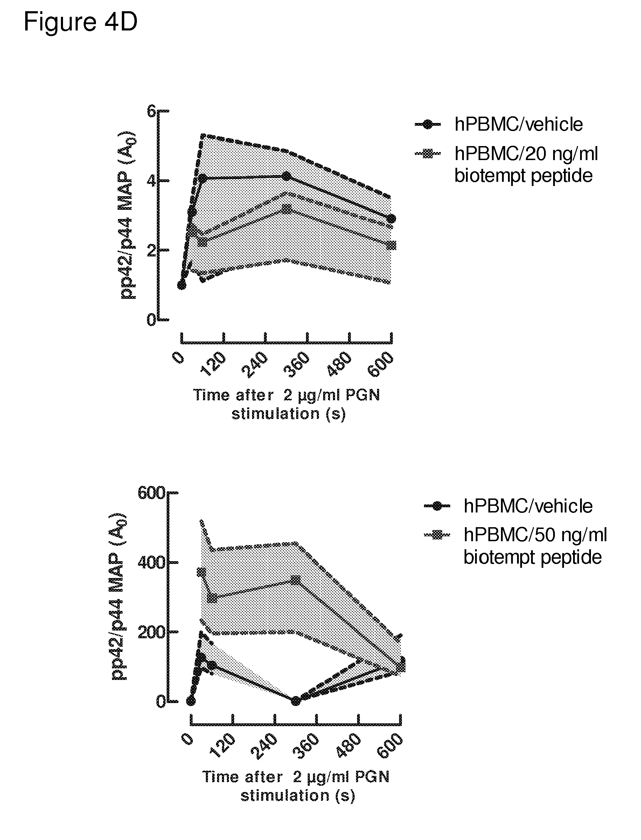

EA-230 seemingly stimulates NOD-signalling induced by peptidoglycan at 50 ng/ml under the conditions tested, as measured by PKB, JNK, p38MAPK and P42/p44MAPK/ERK1,2 phosphorylation status (figure 4).

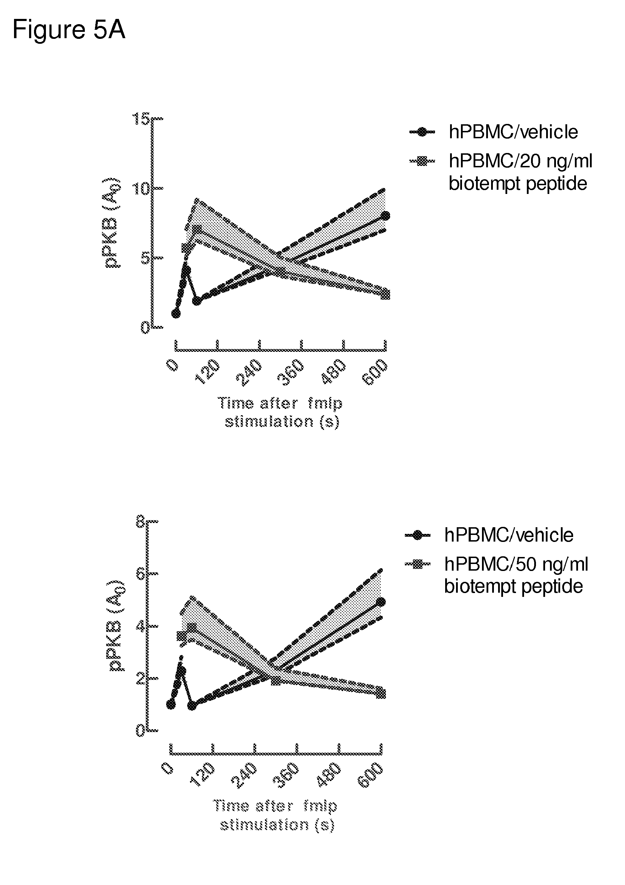

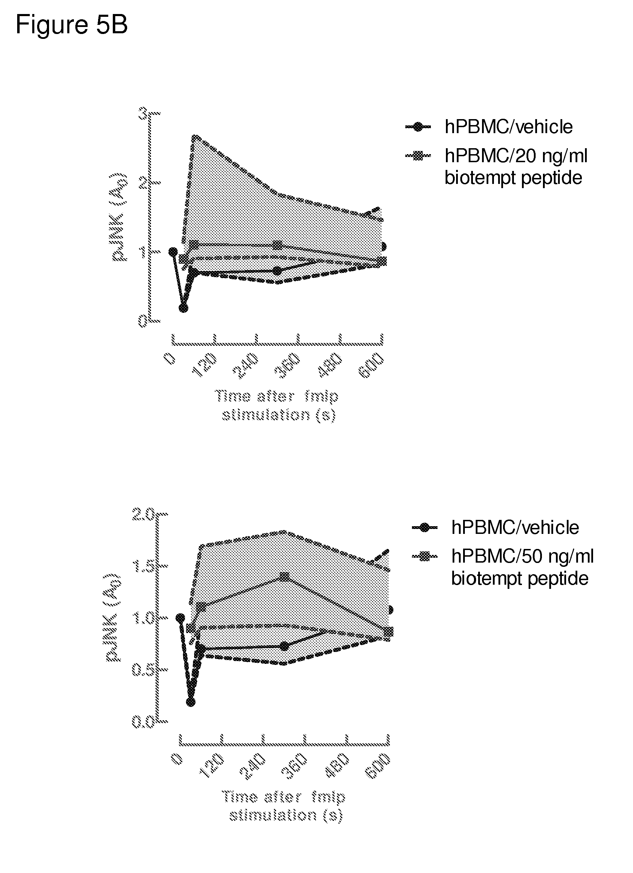

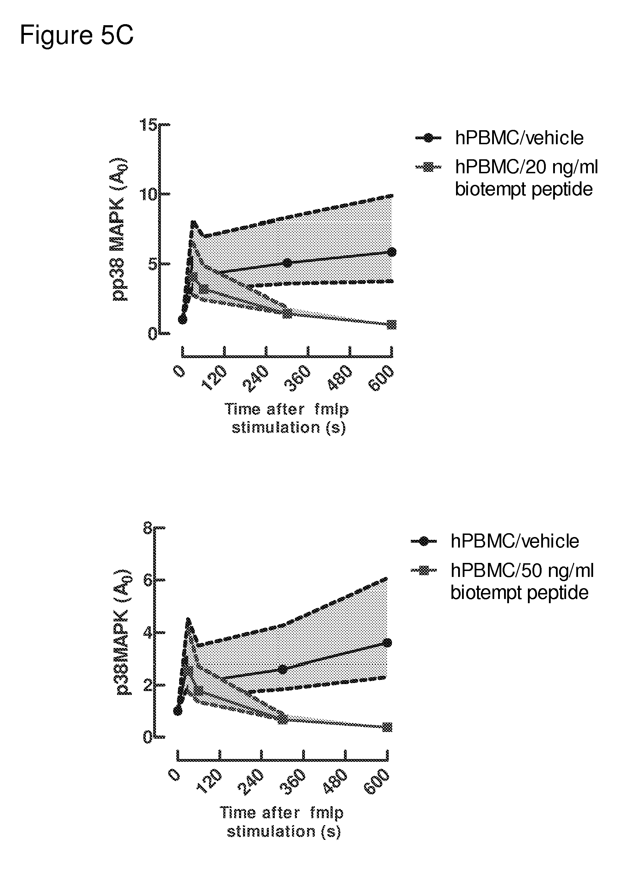

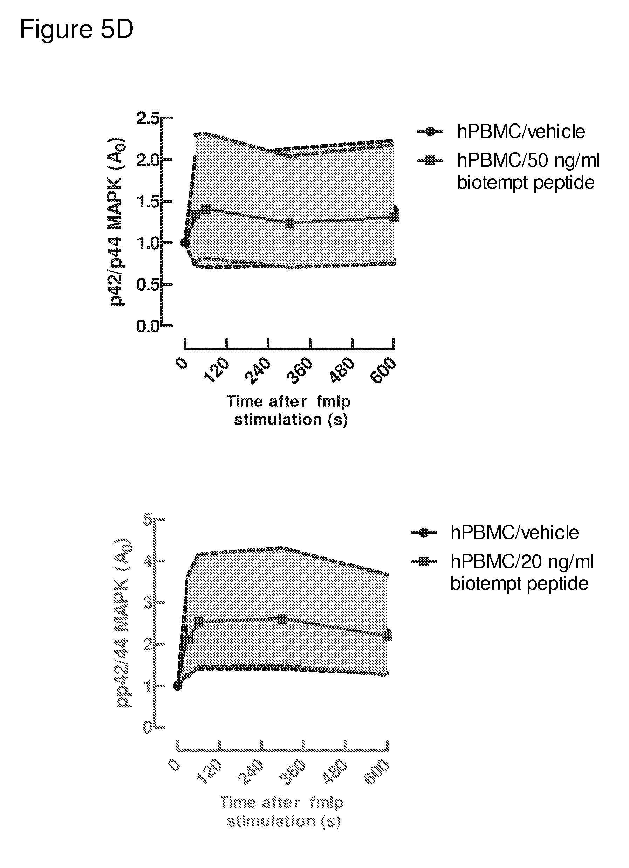

FPR signalling induced by fMLP was slightly attenuated by EA-230 in that induction of PKB phosphorylation was initially seen, followed by downregulation of PKB and p38MAPK phosphorylation when tested at both 20 ng/ml and 50 ng/ml (figure 5).

### References Example 3

¹ van den Blink B, Branger J, Weijer S, Deventer SH, van der Poll T, Peppelenbosch MP. Human endotoxemia activates p38 MAP kinase and p42/44 MAP kinase, but not c-Jun N-terminal kinase. Mol Med. 2001 Nov;7(11):755-60.
² O'Toole T, Peppelenbosch MP. Phosphatidyl inositol-3-phosphate kinase mediates CD14 dependent signaling. Mol Immunol. 2007 Mar;44(9):2362-9.
³ Versteeg HH, Nijhuis E, van den Brink GR, Evertzen M, Pynaert GN, van Deventer SJ, Coffer PJ, Peppelenbosch MP. A new phosphospecific cell-based ELISA for p42/p44 mitogen-activated protein kinase (MAPK), p38 MAPK, protein kinase B and cAMP-response-element-binding protein. Biochem J. 2000 Sep 15;350:717-22.
⁴ Bos CL, Diks SH, Hardwick JC, Walburg KV, Peppelenbosch MP, Richel DJ. Protein phosphatase 2A is required for mesalazine-dependent inhibition of Wnt/beta-catenin pathway activity. Carcinogenesis. 2006 27:2371-82

### Example 4

### Materials and methods

PMBC-derived monocytes were isolated from healthy volunteers (hPBMCs) as in example 3 . Subsequently PMBC were incubated with 1.3, 3.2, 8, 20 and 50 ng/ml peptide AQGV , LQGV, AQG, LQG, QGV, AQ, LQ, GV or QG (peptide, which was freshly prepared as a 20 mg/ml stock solution in bidistilled water) or treated with the cell culture medium alone for 10 min.

hPMBC were challenged with 2 µg/ml TLR-4 ligand LPS for 1 min, 5 min, 10 min and 20 min.

Following stimulation, the phosphorylation status of PKB and p38 MAPK, was analysed as in example 3. Results were corrected for differences using co-measurement of β-actin as in example 3.

### Results

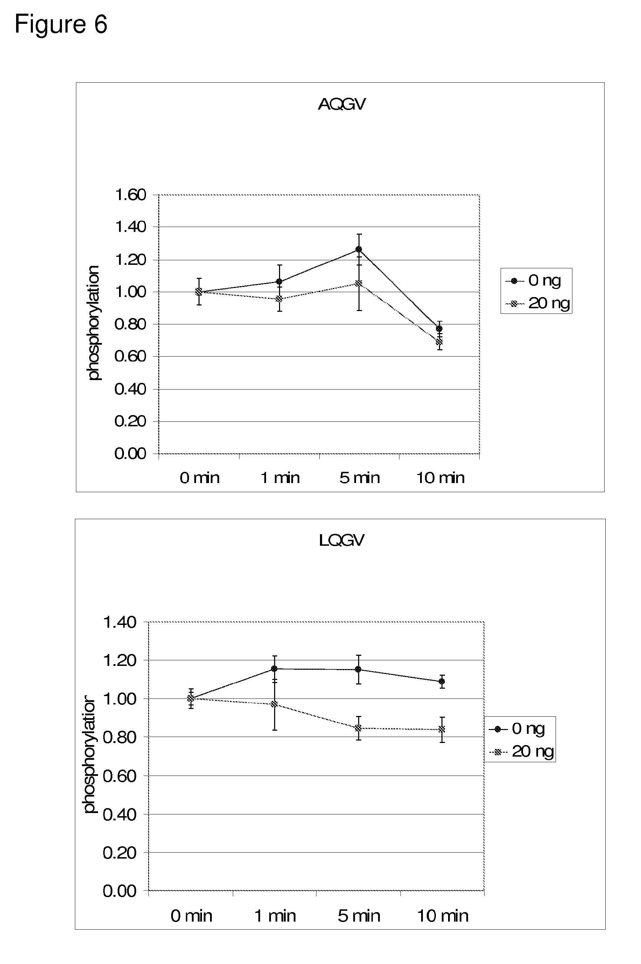

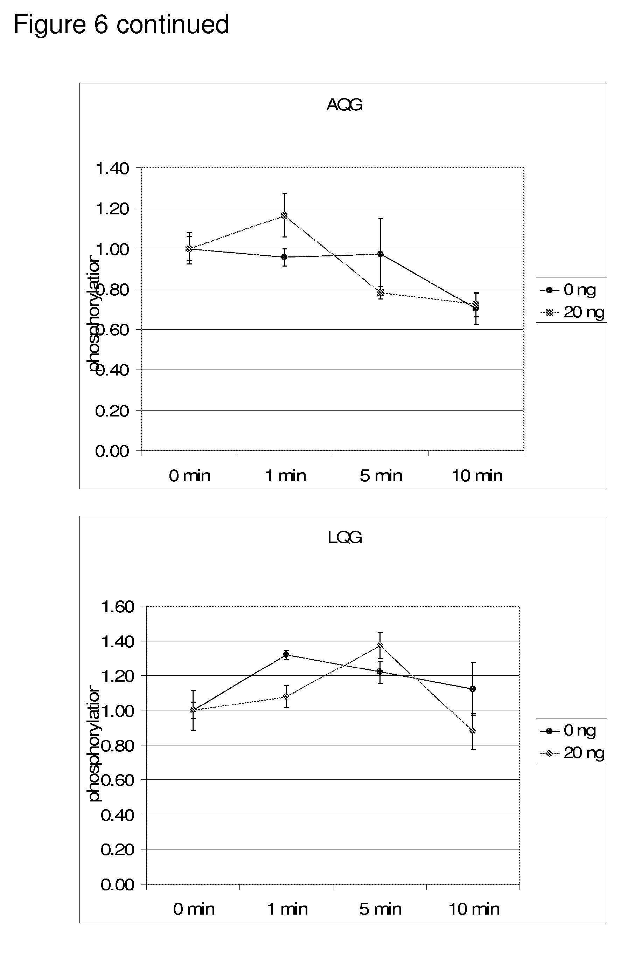

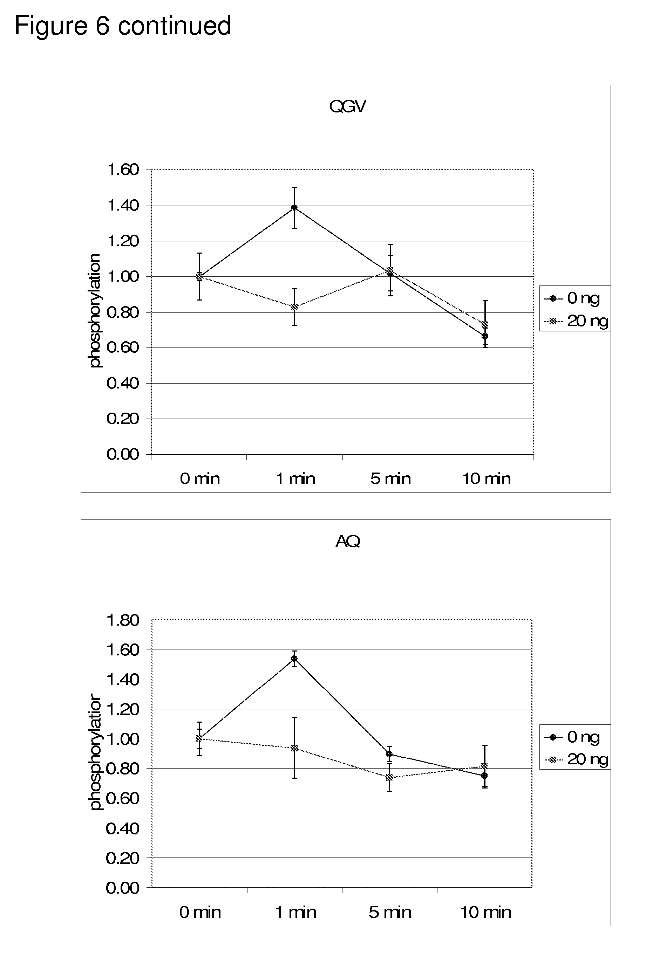

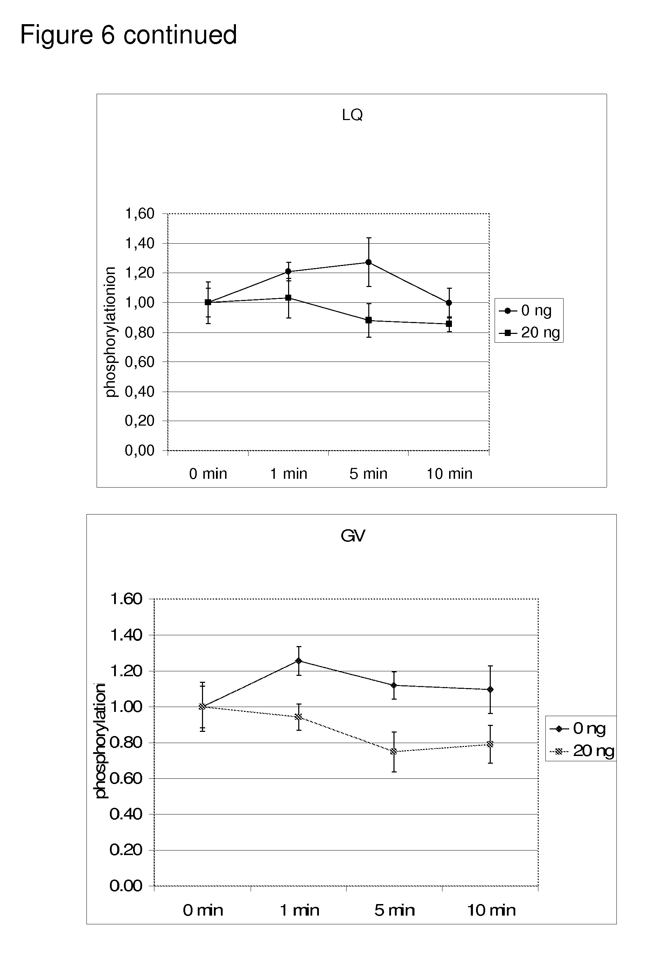

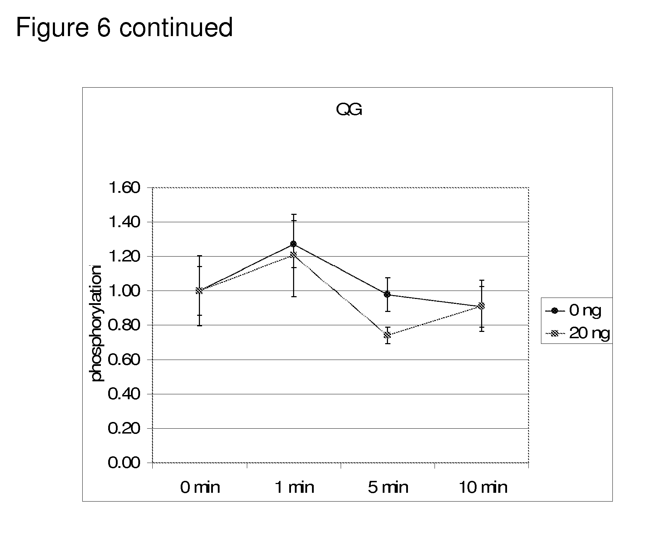

Peptides AQGV and LQGV reduced TLR-4 mediated signalling induced by LPS, as measured by PKB phosphorylation status, when tested at 20 ng/ml (figure 6). Peptide QGV seemingly result in an, at least initial, inhibition of TLR-4 mediated signalling induced by LPS. Surprisingly, dipeptides AQ, LQ and GV, like AQGV and LQGV, inhibited LPS induced TLR-4 mediated signalling.

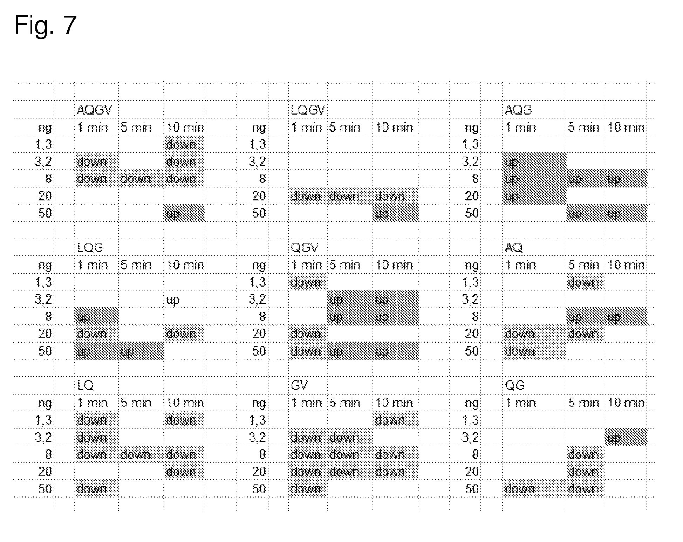

Figure 7 indicates significant upregulation or downregulation of PKB phosphorylation for each concentration of peptide as compared to control (LPS stimulated, no peptide added). Peptides AQGV, QGV, AQ, LQ and GV already display a modulation of TLR-4 mediated signalling induced by LPS, as measured by PKB phosphorylation status, when tested at low concentrations, i.e. below 20 ng/ml. Peptide LQGV significantly downregulated TLR-4 mediated signalling induced by LPS, when tested at 20 ng/ml.

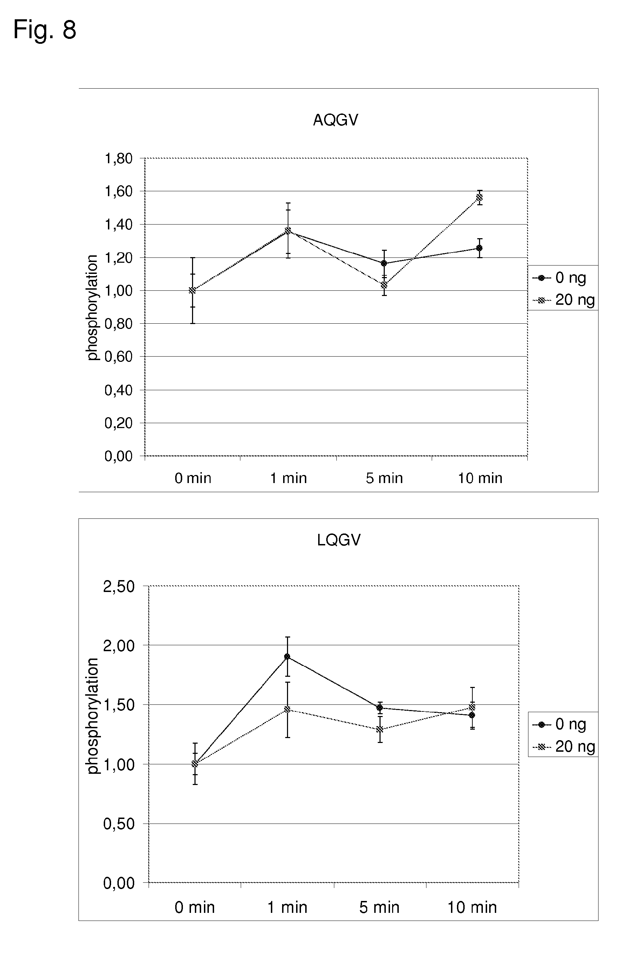

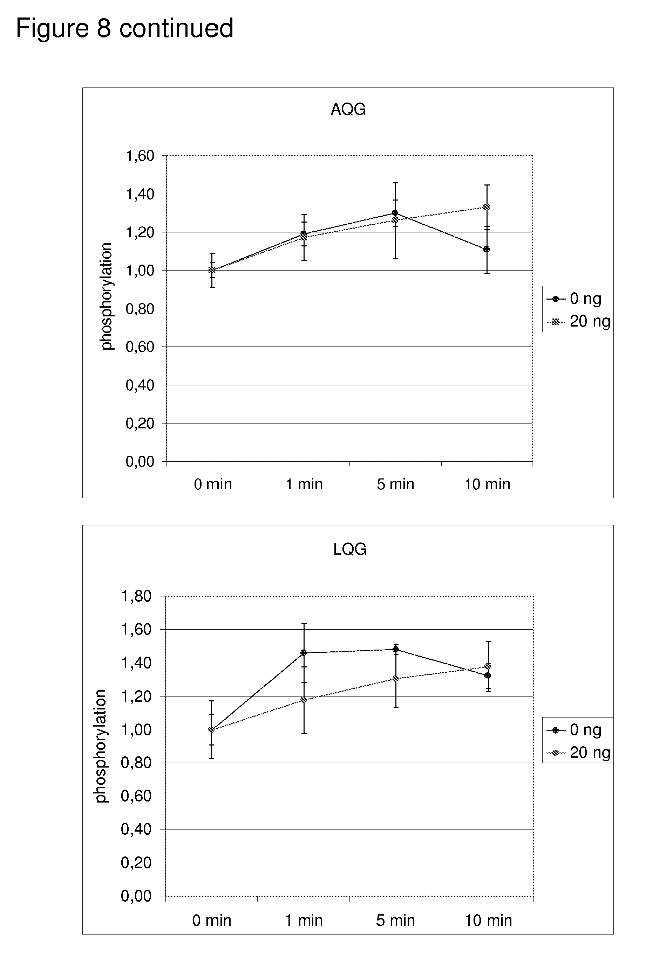

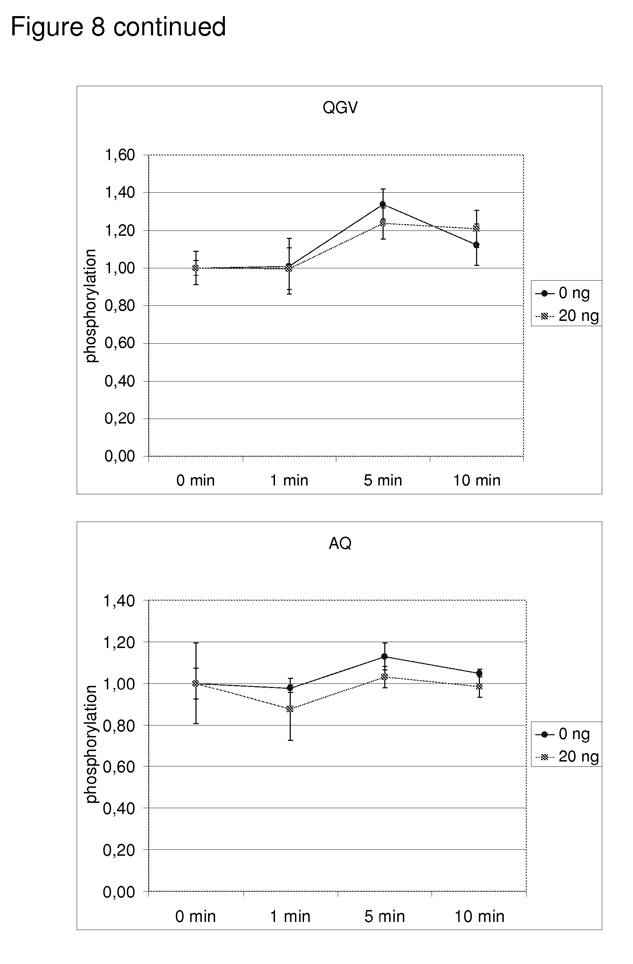

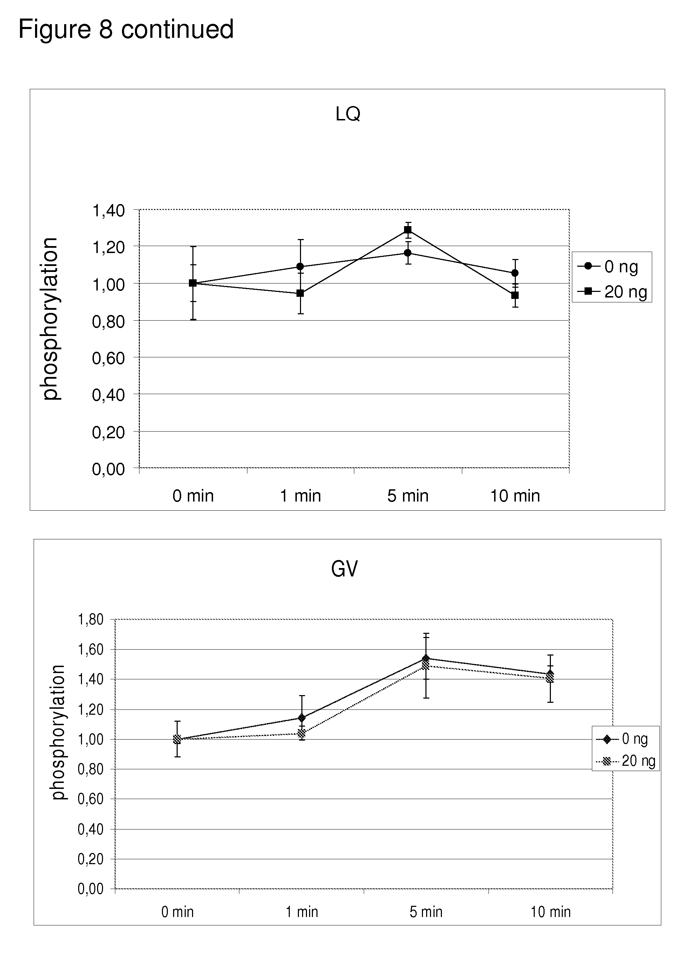

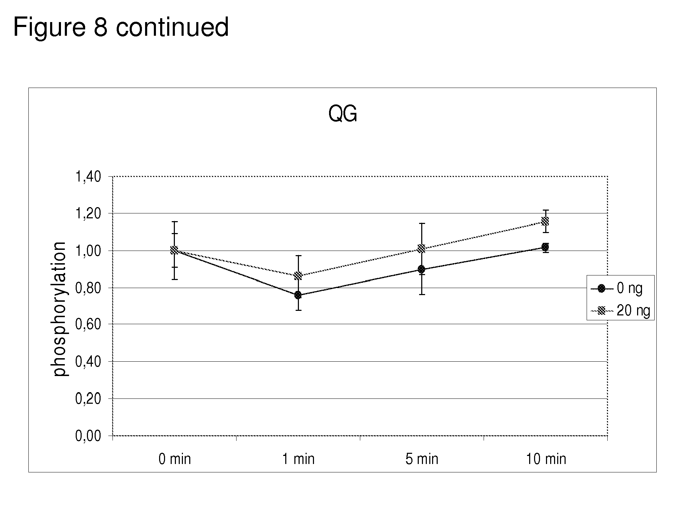

Figure 8 shows the effect of peptides AQGV , LQGV, AQG, LQG, QGV, AQ, LQ, GV and QG on TLR-4 mediated signalling induced by LPS, as measured by p38 MAPK phosphorylation status, when tested at 20 ng/ml. Peptides AQGV, LQGV and LQG show an initial downregulation at this concentration. Peptides AQ and GV demonstrated a slight overall downregulation and peptide QG a slight upregulation of TLR-4 mediated signalling induced by LPS, as measured by p38 MAPK phosphorylation status.

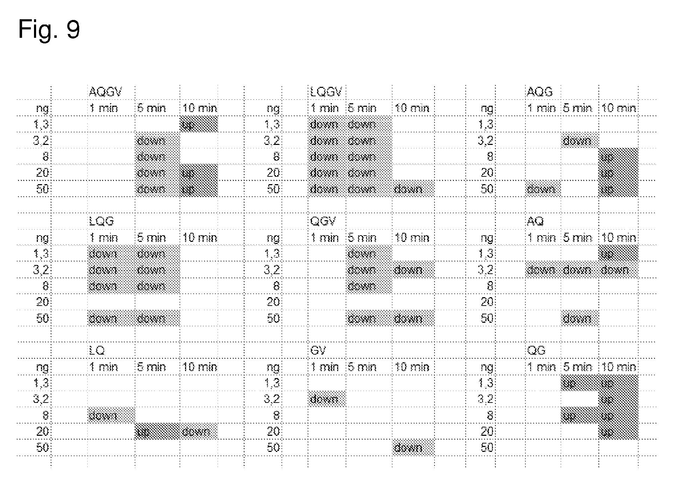

Figure 9 significant upregulation or downregulation of p38 MAPK phosphorylation for each concentration of peptide as compared to control (LPS stimulated, no peptide added). Figure 9 demonstrates that also p38 MAPK phosphorylation is already modulated by peptides AQGV, LQGV, LQG, QGV, AQ, and QG at low concentrations of peptide.

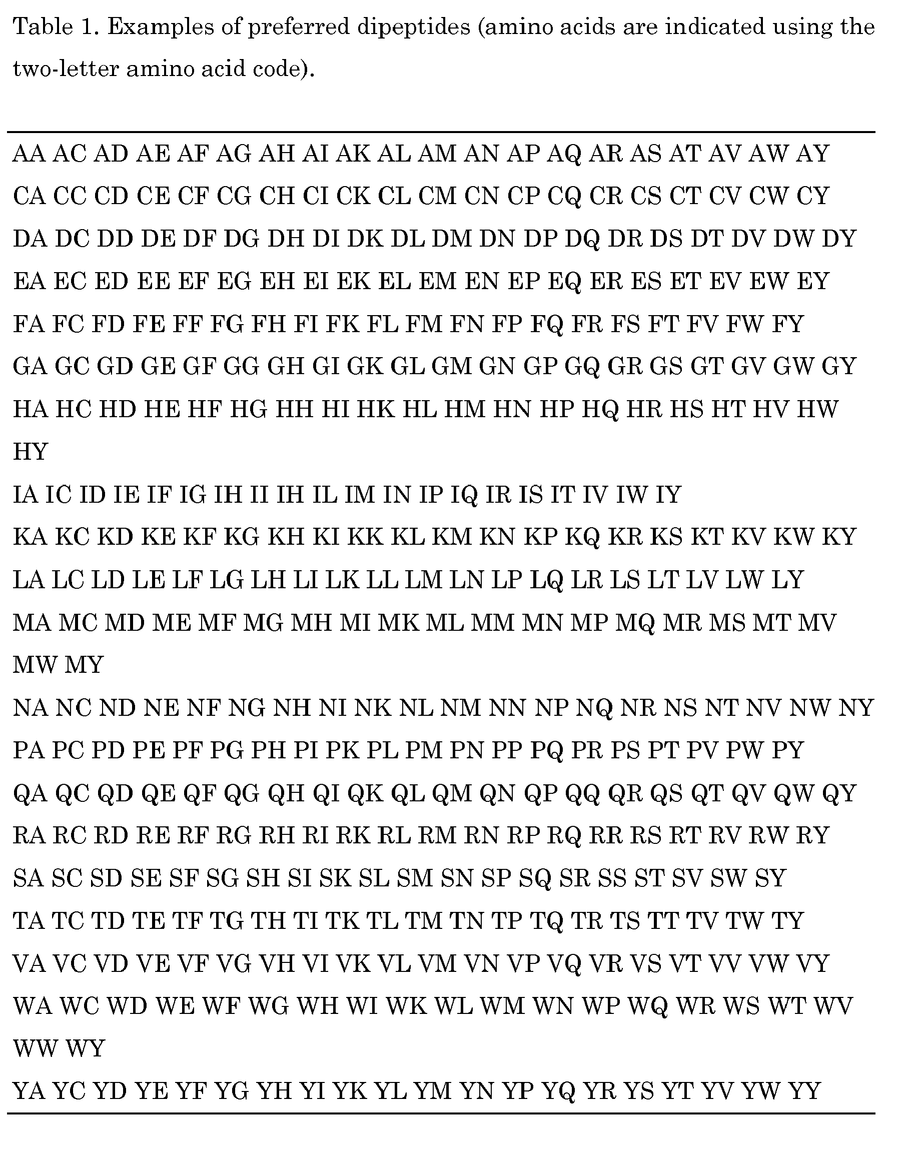

**Table 2. Examples of preferred dipeptides (amino acids are indicated using the two-letter amino acid code)**

| |
|---|
| AA, AG, AL, AP, AQ, AV |
| GA, GG, GL, GP, GQ, GV |
| LA, LG, LL, LP, LQ, LV |
| PA, PG, PL, PP, PQ, PV |
| QA, QG, QL, QP, QQ, QV |
| VA, VG, VL, VP, VQ, VV |
| MT, TR, RV, VL, LQ, QG, |
| GV, LP, PA, AL, PQ |

**Table 3. Examples of preferred dipeptides (amino acids are indicated using the two-letter amino acid code)**

| |
|---|
| AQ, MT, TR, RV, VL, |
| LQ, QG, GV, LP, PA, |
| AL, PQ |

**Table 4. Examples of preferred dipeptides (amino acids are indicated using the two-letter amino acid code)**

| |
|---|
| AQ |
| LQ |
| GV |
| QG |

## Claims

1. A method for determining whether a dipeptide consisting of L-amino acid residues is capable of modulating signalling via at least one Pattern Recognition Receptor (PRR) signalling pathway and/or G protein coupled receptor (GPCR) signalling pathway, the method comprising:
a) providing a cell susceptible to signalling through at least one of said pathways,
b) contacting said cell with at least one dipeptide,
c) contacting said cell with an agonistic or antagonistic compound of a PRR and/or a GPCR of said pathway, and
d) determining the activity of said at least one signalling pathway in said cell.

2. A method according to claim 1 wherein said dipeptide is selected from the group of dipeptides having the general formula H-Xaa1-Xaa2-OH and wherein Xaa1 and Xaa2 are each independently selected from the group of amino acid residues A, C, D, E, F, G, H, I, K, L, M, N, P, Q, R, S, T, V, W and Y.

3. A method according to claim 1 or 2 wherein the amino acid residue at position Xaa1 and/or Xaa2 is not Q.

4. A method according to any one of the preceding claims wherein said cell is contacted with an agonistic or antagonistic compound of a Formyl Peptide Receptor (FPR) signalling pathway.

5. A method according to any one of the preceding claims, wherein said cell is a an antigen presenting cell (APC), preferably a monocyte or a dendritic cell, preferably a PMBC-derived monocyte or dendritic cell, a macrophage, or a granulocyte.

6. A method according to any one of the preceding claims wherein said activity is compared with a reference.

7. A method according to any one of the preceding claims wherein activity of said at least one signalling pathway in said cell is determined by a method comprising determining the activation state of at least one signalling molecule in said pathway.

8. A method according to claim 5 wherein said activation state is determined by measuring the phosphorylation status of said at least one signalling molecule, preferably a protein kinase, more preferably a tyrosine kinase, more preferably selected from the group consisting of PKB/Akt, PI3K, JNK, p38MAPK and p42/p44MAPK/ERK, or a transcription factor, preferably a STAT-family member, more preferably STAT3, or CREB.

9. A method according to claim 7 or 8 wherein said activation state is determined by measuring NF-κB activity.

10. A method according to any one of the preceding claims wherein activity of said at least one signalling pathway in said cell is determined by a method comprising measuring production of inflammatory cytokines.

11. A method according to any one of the preceding claims, wherein said modulating signalling is inhibiting signalling or potentiating signalling.

12. A method according to any one of the preceding claims, wherein said cell is pretreated with at least one of said dipeptides, followed by contacting said cell with said agonistic or antagonistic compound of a PRR and/or a GPCR of said pathway.

13. A method according to any one of the preceding claims, comprising determining whether a dipeptide is capable of modulating signalling via at least two distinct PRR and/or GPCR signalling pathways, preferably two or more signalling pathways selected from the group consisting of the NOD induced signalling pathway, TLR-3 induced signalling pathway, TLR-4 induced signalling pathway, TLR-5 induced signalling pathway, fMLPR induced signalling pathway.

14. A dipeptide consisting of L-amino acid residues for use in modulating a PRR signalling pathway and/or a GPCR signalling pathway, preferably wherein said dipeptide is selected from the group of 400 naturally occurring dipeptides having the general formula H-X₁-X₂-OH and wherein X₁ and X₂ are each independently selected from the group of L-amino acids A, C, D, E, F, G, H, I, K, L, M, N, P, Q, R, S, T, V, W and Y.

15. A dipeptide consisting of L-amino acid residues for use in the treatment or prevention of infection with a pathogen, preferably wherein said dipeptide is selected from the group of 400 naturally occurring dipeptides having the general formula H-X₁-X₂-OH and wherein X₁ and X₂ are each independently selected from the group of L-amino acids A, C, D, E, F, G, H, I, K, L, M, N, P, Q, R, S, T, V, W and Y.

16. A dipeptide according to claim 13 or 14 which is selected from table 1, preferably table 2, more preferably table 3, even more preferably table 4, most preferably wherein said dipeptide is dipeptide GV.
